Europäisches Patentamt
European Patent Office
Office européen des brevets

(19) (11) Veröffentlichungsnummer: **0 285 978 A2**

# EUROPÄISCHE PATENTANMELDUNG

(12)

(21) Anmeldenummer: 88105046.2

(22) Anmeldetag: 29.03.88

(51) Int. Cl.4: C07D 239/42 , C07D 239/47 , C07D 239/48 , C07D 405/12 , C07D 401/12 , C07D 417/12 , C07D 239/56 , C07D 239/38 , A01N 43/54

(30) Priorität: 10.04.87 DE 3712306

(43) Veröffentlichungstag der Anmeldung: 12.10.88 Patentblatt 88/41

(84) Benannte Vertragsstaaten: BE CH DE FR GB IT LI NL

(71) Anmelder: BAYER AG
Konzernverwaltung RP Patentabteilung
D-5090 Leverkusen 1 Bayerwerk(DE)

(72) Erfinder: Pfister, Theodor, Dr.
Lichtenberger Strasse 30
D-4019 Monheim(DE)
Erfinder: Müller, Klaus-Helmut, Dr.
Bockhackstrasse 55
D-4000 Düsseldorf 13(DE)
Erfinder: Santel, Hans-Joachim, Dr.
Grünstrasse 9a
D-5090 Leverkusen 1(DE)
Erfinder: Schmidt, Robert R., Dr.
Im Waldwinkel 110
D-5060 Bergisch Gladbach 2(DE)
Erfinder: Strang, Harry, Dr.
Unterdorfstrasse 6A
D-4000 Düsseldorf 31(DE)

(54) **Substituierte 1-(2-Oxyaminosulfonyl-phenylsulfonyl)-3-(4-trifluormethyl-pyrimidin-2-yl)-(thio)harnstoffe.**

(57) Die Erfindung betrifft neue substituierte 1-(2-Oxyaminosulfonyl-phenylsulfonyl)-3-(4-trifluormethyl-pyrimidin-2-yl)-(thio)harnstoffe der allgemeinen Formel (I)

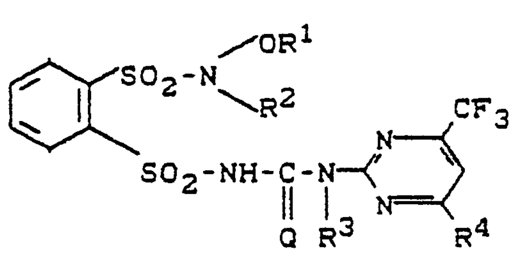

(I)

in welcher
Q für Sauerstoff oder Schwefel steht und die Reste $R^1$, $R^2$, $R^3$ und $R^4$ die in der Beschreibung angegebenen Bedeutungen haben,

EP 0 285 978 A2

verschiedene Verfahren und neue Zwischenprodukte zu ihrer Herstellung und ihre Verwendung als Herbizide.

# Substituierte 1-(2-Oxyaminosulfonyl-phenylsulfonyl)-3-(4-trifluormethyl-pyrimidin-2-yl)-(thio)harnstoffe

Die Erfindung betrifft neue substituierte 1-(2-Oxyaminosulfonyl-phenylsulfonyl)-3-(4-trifluormethyl-pyrimidin-2-yl)-(thio)harnstoffe, Verfahren und neue Zwischenprodukte zu ihrer Herstellung sowie ihre Verwendung als Herbizide.

Es ist bekannt, daß bestimmte substituierte 1-Phenylsulfonyl-3-pyrimidinyl-harnstoffe, wie z. B. 1-(2-Methoxyphenylsulfonyl)-3-(4,6-dimethyl-pyrimidin-2-yl)-harnstoff, herbizid wirksam sind (vgl. US-PS 4 169 719). Die Wirkung dieser Verbindungen ist jedoch nicht immer ganz befriedigend.

Es wurden nun neue substituierte 1-(2-Oxyaminosulfonylphenylsulfonyl)-3-(4-trifluormethyl-pyrimidin-2-yl)-(thio)harnstoffe der allgemeinen Formel (I)

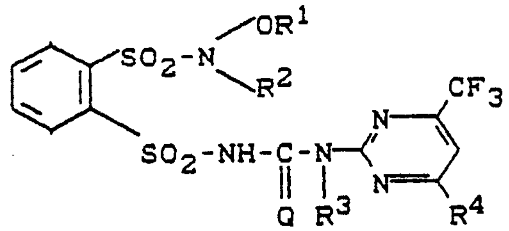

(I)

in welcher

Q für Sauerstoff oder Schwefel steht,

$R^1$ für $C_1$-$C_8$-Alkyl [welches gegebenenfalls durch Fluor, Chlor, Cyano, $C_1$-$C_2$-Alkoxy, $C_1$-$C_2$-Alkylthio, $C_1$-$C_2$-Alkylsulfinyl oder $C_1$-$C_2$-Alkylsulfonyl substituiert ist], für $C_3$-$C_6$-Alkenyl [welches gegebenenfalls durch Fluor oder Chlor substituiert ist], für $C_3$-$C_6$-Alkinyl, $C_3$-$C_6$-Cycloalkyl, $C_3$-$C_6$-Cycloalkyl-$C_1$-$C_2$-alkyl oder Phenyl-$C_1$-$C_2$-alkyl [welches gegebenenfalls durch Fluor, Chlor, Nitro, Cyano, $C_1$-$C_2$-Alkyl, $C_1$-$C_2$-Alkoxy oder $C_1$-$C_2$-Alkoxy-carbonyl substituiert ist] steht,

$R^2$ für Wasserstoff, für $C_1$-$C_8$-Alkyl [welches gegebenenfalls durch Fluor, Chlor, Cyano, $C_1$-$C_2$-Alkoxy, $C_1$-$C_2$-Alkylthio, $C_1$-$C_2$-Alkylsulfinyl, $C_1$-$C_4$-Alkylsulfonyl oder $C_1$-$C_2$-Alkoxy-carbonyl substituiert ist], für $C_3$-$C_6$-Alkenyl, $C_3$-$C_6$-Alkinyl oder Phenyl-$C_1$-$C_2$-alkyl [welches gegebenenfalls durch Fluor, Chlor, Nitro, Cyano, $C_1$-$C_2$-Alkyl, $C_1$-$C_2$-Alkoxy oder $C_1$-$C_2$-Alkoxy-carbonyl substituiert ist] steht, oder zusammen mit $R^1$ für $C_3$-$C_5$-Alkandiyl (Alkylen) steht, oder für den Rest -CO-$R^5$ steht, worin

$R^5$ für Wasserstoff, $C_1$-$C_{10}$-Alkyl [welches gegebenenfalls durch Fluor, Chlor, Brom, Cyano, $C_1$-$C_2$-Alkoxy-carbonyl, $C_1$-$C_2$-Alkoxy, Benzyloxy oder durch gegebenenfalls durch Fluor, Chlor, Brom, Cyano, Nitro, Methyl und/oder Trifluormethyl substituiertes Phenoxy substituiert ist], $C_3$-$C_6$-Cycloalkyl, $C_3$-$C_6$-Cycloalkyl-$C_1$-$C_2$-alkyl, $C_2$-$C_6$-Alkenyl [welches gegebenenfalls durch Fluor, Chlor oder Brom substituiert ist], $C_2$-$C_6$-Alkinyl, Phenyl-$C_1$-$C_2$-alkyl [welches gegebenenfalls durch Fluor, Chlor, Nitro, Cyano, $C_1$-$C_2$-Alkyl, $C_1$-$C_2$-Alkoxy oder $C_1$-$C_2$-Alkoxy-carbonyl substituiert ist], Phenyl [welches gegebenenfalls durch Fluor, Chlor, Cyano, Nitro, $C_1$-$C_2$-Alkyl, Trifluormethyl, $C_1$-$C_2$-Alkoxy, $C_1$-$C_2$-Fluoralkoxy oder $C_1$-$C_2$-Alkoxy-carbonyl substituiert ist], Pyridyl [welches gegebenenfalls durch Fluor, Chlor, Cyano, Nitro, $C_1$-$C_2$-Alkyl oder $C_1$-$C_2$-Alkoxy substituiert ist], Furyl [welches gegebenenfalls durch Fluor, Chlor, Cyano, Nitro, $C_1$-$C_2$-Alkyl oder $C_1$-$C_2$-Alkoxy substituiert ist], $C_1$-$C_6$-Alkoxy [welches gegebenenfalls durch Fluor, Chlor, Cyano oder $C_1$-$C_2$-Alkoxy substituiert ist], Benzyloxy [welches gegebenenfalls durch Fluor, Chlor, Nitro, Cyano, $C_1$-$C_2$-Alkyl, $C_1$-$C_2$-Alkoxy oder $C_1$-$C_2$-Alkoxy-carbonyl substituiert ist], Phenoxy [welches gegebenenfalls durch Fluor, Chlor, Cyano, Nitro, $C_1$-$C_2$-Alkyl, Trifluormethyl, $C_1$-$C_2$-Alkoxy, $C_1$-$C_2$-Fluoralkoxy oder $C_1$-$C_2$-Alkoxy-carbonyl substituiert ist] oder $C_1$-$C_2$-Alkoxy-carbonyl steht, in welcher weiter

$R^3$ für Wasserstoff, ein Alkalimetall-oder ein Erdalkalimetall-äquivalent, ein Ammonium-äquivalent, ein $C_1$-$C_4$-Alkyl-ammonium-äquivalent [welches gegebenenfalls durch Fluor, Chlor oder Hydroxy substituiert ist], ein Di-($C_1$-$C_4$)-, Tri-($C_1$-$C_4$)-oder Tetra-($C_1$-$C_4$)-alkylammonium-äquivalent oder ein Guanidiniumäquivalent, für $C_1$-$C_8$-Alkyl [welches gegebenenfalls durch Fluor, Chlor, Cyano, $C_1$-$C_2$-Alkoxy, $C_1$-$C_2$-Alkylthio, $C_1$-$C_2$-Alkylsulfinyl, $C_1$-$C_2$-Alkylsulfonyl oder $C_1$-$C_2$-Alkoxy-carbonyl substituiert ist], für $C_3$-$C_6$-Alkenyl, $C_3$-$C_6$-Alkinyl oder Phenyl-$C_1$-$C_2$-alkyl [welches gegebenenfalls durch Fluor, Chlor, Nitro, Cyano, $C_1$-$C_2$-Alkyl, $C_1$-$C_2$-Alkoxy oder $C_1$-$C_2$-Alkoxy-carbonyl substituiert ist] steht und

$R^4$ für Wasserstoff, Fluor, Chlor, Brom, $C_1$-$C_4$-Alkyl [welches gegebenenfalls durch Fluor und/oder Chlor

substituiert ist], $C_1$-$C_4$-Alkoxy [welches gegebenenfalls durch Fluor, Chlor oder $C_1$-$C_2$-Alkoxy substituiert ist], $C_1$-$C_4$-Alkylamino, $C_1$-$C_4$-Alkoxyamino, N-($C_1$-$C_4$-Alkyl)-O-($C_1$-$C_4$-alkyl)-oxyamino oder Di-($C_1$-$C_2$-alkyl)-amino steht,
gefunden.

Man erhält die neuen Verbindungen der Formel (I), wenn man

(a) Benzodisultame der allgemeinen Formel (II)

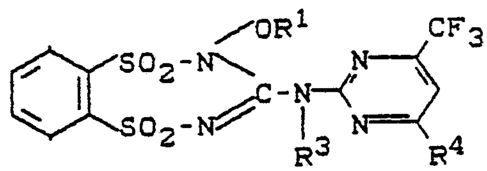

(II)

in welcher
$R^1$, $R^3$ und $R^4$ die oben angegebenen Bedeutungen haben,
mit Wasser oder Schwefelwasserstoff, gegebenenfalls in Gegenwart eines Katalysators und gegebenenfalls in Gegenwart eines Verdünnungsmittels, umsetzt, oder

(b) Oxyguanidine der allgemeinen Formel (III)

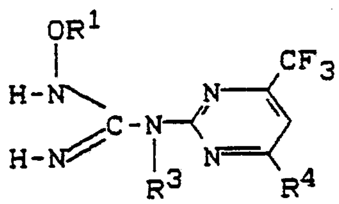

(III)

in welcher
$R^1$, $R^3$ und $R^4$ die oben angegebenen Bedeutungen haben,
mit Benzol-1,2-disulfonsäure-dichlorid, gegebenenfalls in Gegenwart eines Säureakzeptors und gegebenenfalls in Gegenwart eines Verdünnungsmittels, umsetzt und die hierbei erhaltenen Benzodisultame der Formel (II) - ohne Zwischenisolierung - mit Wasser oder mit Schwefelwasserstoff, gegebenenfalls in Gegenwart eines Katalysators und gegebenenfalls in Gegenwart eines Verdünnungsmittels umsetzt, oder

(c) 2-Amino-4-trifluormethyl-pyrimidine der allgemeinen Formel (IV)

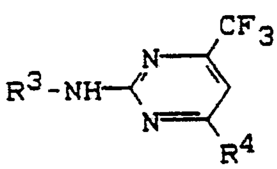

(IV)

in welcher
$R^3$ und $R^4$ die oben angegebenen Bedeutungen haben,
mit 2-Oxyaminosulfonyl-phenylsulfonyl-iso(thio)cyanaten der allgemeinen Formel (V)

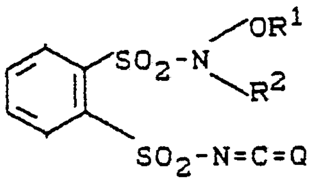

(V)

in welcher
Q, $R^1$ und $R^2$ die oben angegebenen Bedeutungen haben,
gegebenenfalls in Gegenwart eines Verdünnungsmittels und gegebenenfalls in Gegenwart eines Katalysators umsetzt, oder

(d) 1-(2-Oxyaminosulfonyl-phenylsulfonyl)-3-(4-trifluormethyl-pyrimidin-2-yl)-(thio)harnstoffe der allgemeinen Formel (VI)

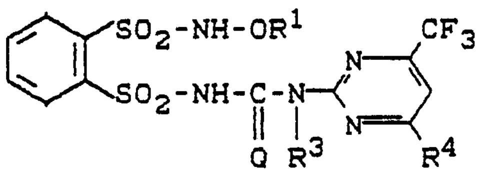

(VI)

in welcher
Q, $R^1$, $R^3$ und $R^4$ die oben angegebenen Bedeutungen haben,
mit Verbindungen der Formel (VII)

$R^2$ -X (VII)

in welcher
$R^2$ die oben angegebene Bedeutung hat und
X für Halogen steht,
gegebenenfalls in Gegenwart eines Säureakzeptors und gegebenenfalls in Gegenwart eines Verdünnungsmittels umsetzt, und gegebenenfalls die gemäß Verfahren (a), (b), (c) oder (d) erhaltenen Verbindungen der Formel (I), in welcher $R^3$ für Wasserstoff steht, nach üblichen Methoden in Salze überführt.

Die neuen substituierten 1-(2-Oxyaminosulfonyl-phenylsulfonyl)-3-(4-trifluormethyl-pyrimidin-2-yl)-(thio)-harnstoffe der allgemeinen Formel (I) zeichnen sich durch starke herbizide Wirksamkeit aus.

Überraschenderweise zeigen die neuen Verbindungen der Formel (I) erheblich bessere herbizide Wirkung als vorbekannte 1-Arylsulfonyl-3-heteroaryl-(thio)harnstoffe gleicher Wirkungsrichtung.

Weitere mögliche Herstellungsmethoden für die erfindungsgemäßen Verbindungen der Formel (I) sind nachstehend aufgeführt, wobei Q, $R^1$, $R^2$, $R^3$ und $R^4$ die oben angegebenen Bedeutungen haben:

(e) Umsetzung von 2-Oxyaminosulfonyl-benzolsulfonsäureamiden (VIII) mit N-(4-Trifluormethyl-pyrimidin-2-yl)-(thio)urethanen (IX) ($R^6$ : Methyl, Ethyl, Benzyl, Phenyl):

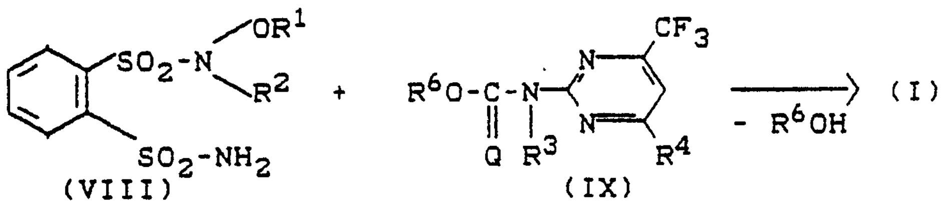

(f) Umsetzung von N-(2-Oxyaminosulfonyl-phenylsulfonyl)-(thio)urethanen (X) ($R^7$ : Methyl, Ethyl, Ben zyl, Phenyl) mit 2-Amino-4-trifluormethyl-pyrimidinen (IV):

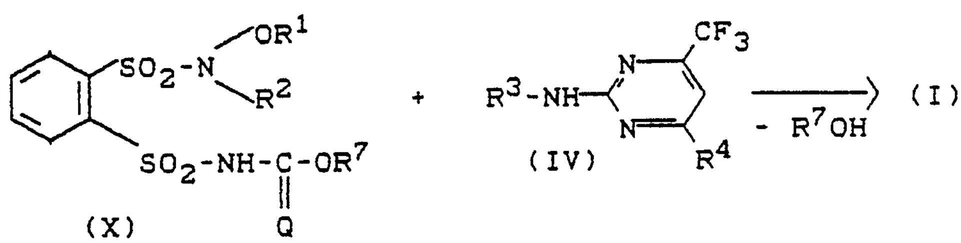

(g) Umsetzung von 1-(2-Oxyaminosulfonyl-phenylsulfonyl)-3-(4-trifluormethyl-pyrimidin-2-yl)-(thio)-harnstoffen (XI) mit "Alkylierungsmitteln" (XII) (W : nucleofuge Abgangsgruppe):

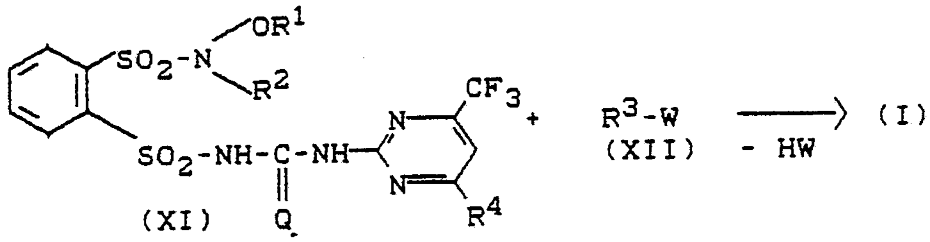

(h) Umsetzung von 2-Fluorsulfonyl-phenyliso(thio)cyanat mit 2-Amino-4-trifluormethyl-pyrimidinen (IV) und weiter mit Hydroxylamin-Derivaten:

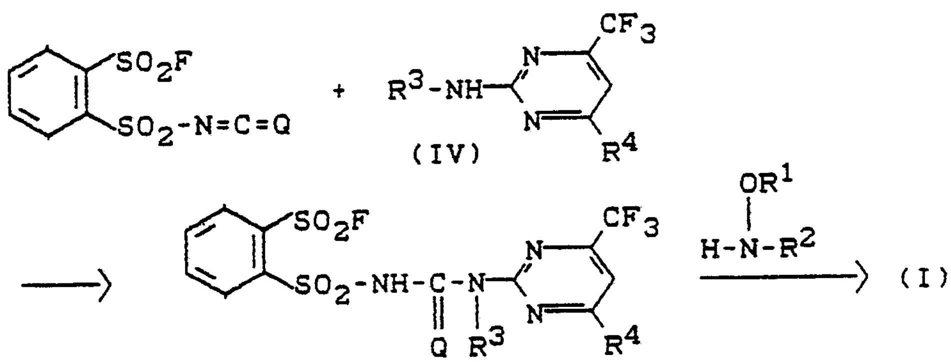

Gegenstand der Erfindung sind vorzugsweise Verbindungen der Formel (I), in welcher
Q für Sauerstoff oder Schwefel steht,
$R^1$ für $C_1$-$C_4$-Alkyl, $C_3$-$C_4$-Alkenyl oder $C_3$-$C_4$-Alkinyl steht,
$R^2$ für Wasserstoff, $C_1$-$C_4$-Alkyl, $C_3$-$C_4$-Alkenyl, $C_3$-$C_4$-Alkinyl oder zusammen mit $R^1$ für $C_3$-$C_4$-Alkandiyl (Trimethylen oder Tetramethylen) steht, oder für den Rest -CO-$R^5$ steht, worin
$R^5$ für Wasserstoff, $C_1$-$C_4$-Alkyl [welches gegebenenfalls durch Fluor oder Chlor substituiert ist], Cyclopropyl, $C_2$-$C_4$-Alkenyl [welches gegebenenfalls durch Fluor oder Chlor substituiert ist], Benzyl, Phenyl [welches gegebenenfalls durch Fluor, Chlor, Nitro, Methyl, Trifluormethyl, Methoxy oder Trifluormethoxy substituiert ist] Pyridyl oder Furyl steht, in welcher weiter
$R^3$ für Wasserstoff, ein Natrium-, Kalium-, Calcium-oder Ammonium-äquivalent, ein $C_1$-$C_3$-Alkyl-, Di-($C_1$-$C_3$)-, Tri-($C_1$-$C_3$)-oder Tetra-($C_1$-$C_3$)-alkylammonium-äquivalent oder für Methyl steht, und
$R^4$ für Wasserstoff, Chlor, Methyl, Trifluormethyl, Methoxy, Ethoxy, Propoxy, Isopropoxy, 2,2,2-Tri fluorethoxy, Difluormethoxy, Methylamino, Ethylamino oder Dimethylamino steht.

Gegenstand der Erfindung sind insbesondere Verbindungen der Formel (I), in welcher
Q für Sauerstoff steht,
$R^1$ für Methyl, Ethyl oder Allyl steht,
$R^2$ für Wasserstoff oder für den Rest -CO-$R^5$ steht, worin
$R^5$ für $C_1$-$C_3$-Alkyl [welches gegebenenfalls durch Fluor oder Chlor substituiert ist], Cyclopropyl, Benzyl, Phenyl oder Furyl steht,
$R^3$ für Wasserstoff steht und
$R^4$ für Methoxy, Ethoxy oder Difluormethoxy steht.

Die bei dem oben unter (a) angegebenen erfindungsgemäßen Herstellungsverfahren ablaufende chemische Reaktion kann beispielsweise durch folgendes Formelschema skizziert werden:

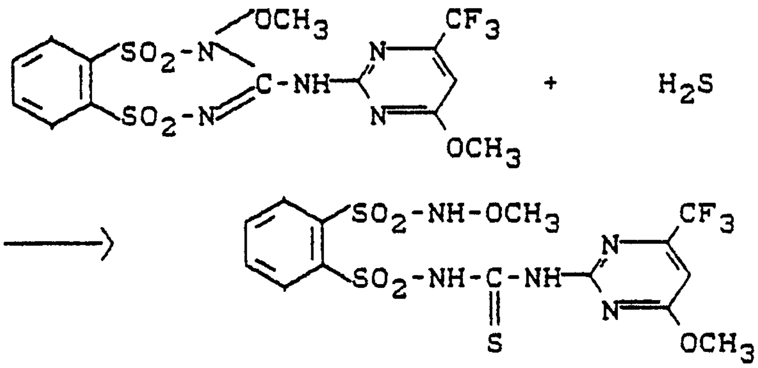

Die bei dem oben unter (b) angegebenen erfindungsgemäßen Herstellungsverfahren ablaufenden chemischen Reaktionen können beispielsweise durch folgendes Formelschema skizziert werden:

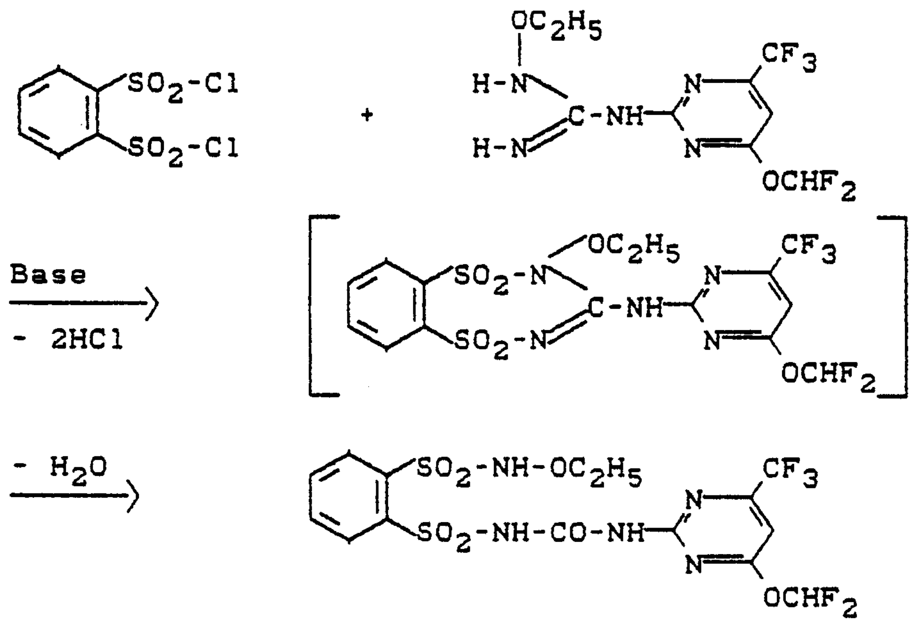


Die bei dem oben unter (c) angegebenen erfindungsgemäßen Herstellungsverfahren ablaufende chemische Reaktion kann beispielsweise durch folgendes Formelschema skizziert werden:

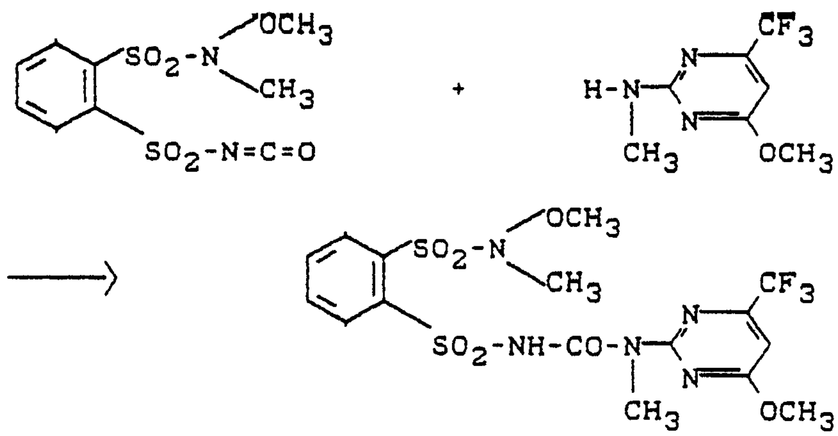

Die bei dem oben unter (d) angegebenen erfindungsgemäßen Herstellungsverfahren ablaufende chemische Reaktion kann beispielsweise durch folgendes Formelschema skizziert werden:

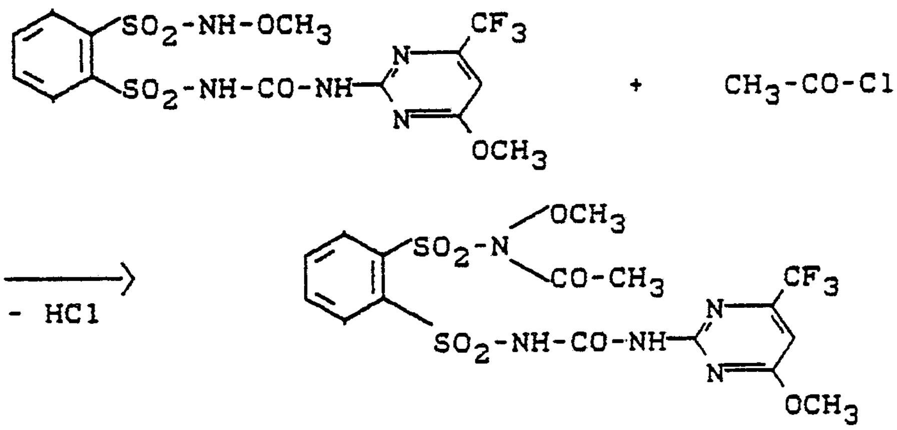


Die beim erfindungsgemäßen Verfahren (a) als Ausgangsstoffe zu verwendenden Benzodisultame sind durch die Formel (II) allgemein definiert.

In Formel (II) haben $R^1$, $R^3$ und $R^4$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Beispiele für die Ausgangsstoffe der Formel (II) sind in der nachstehenden Tabelle 1 aufgeführt.

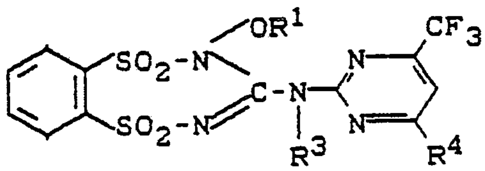

(II)

Tabelle 1: Beispiele für Ausgangssstoffe der Formel (II)

| $R^1$ | $R^3$ | $R^4$ | $R^1$ | $R^3$ | $R^4$ |
|---|---|---|---|---|---|
| $CH_3$ | H | F | $CH_3$ | H | H |
| $CH_3$ | H | Cl | $-CH_2-CH=CH_2$ | H | Cl |
| $CH_3$ | H | Br | $-CH_2-CH=CH_2$ | H | $CF_3$ |
| $CH_3$ | H | $CH_3$ | $-CH_2-CH=CH_2$ | H | $CH_3$ |
| $CH_3$ | H | $CF_3$ | $-CH_2-CH=CH_2$ | H | $OCH_3$ |
| $CH_3$ | H | $OCH_3$ | $-CH_2-CH=CH_2$ | H | $N(CH_3)_2$ |
| $CH_3$ | H | $OC_2H_5$ | $-CH_2-CH=CH_2$ | H | $OC_2H_5$ |
| $CH_3$ | H | $OCHF_2$ | $-CH_2-CH=CH_2$ | H | $OCHF_2$ |
| $CH_3$ | H | $NHCH_3$ | $CH(CH_3)_2$ | H | $OCH_3$ |
| $CH_3$ | H | $O-C_3H_7-n$ | $CH(CH_3)_2$ | H | $OCHF_2$ |
| $CH_3$ | H | $O-C_3H_7-i$ | $C_2H_5$ | H | H |
| $CH_3$ | H | $NHC_2H_5$ | | | |
| $CH_3$ | H | $N(CH_3)_2$ | | | |
| $CH_3$ | $CH_3$ | Cl | | | |
| $CH_3$ | $CH_3$ | $CF_3$ | | | |
| $CH_3$ | $CH_3$ | $CH_3$ | | | |
| $CH_3$ | $CH_3$ | $OCH_3$ | | | |
| $CH_3$ | $CH_3$ | $OC_2H_5$ | | | |
| $CH_3$ | $CH_3$ | $OCHF_2$ | | | |
| $CH_3$ | $CH_3$ | $NHCH_3$ | | | |
| $C_2H_5$ | H | Cl | | | |
| $C_2H_5$ | H | $CF_3$ | | | |
| $C_2H_5$ | H | $OCH_3$ | | | |
| $C_2H_5$ | H | $OC_2H_5$ | | | |
| $C_2H_5$ | H | $OCHF_2$ | | | |
| $C_3H_7$ | H | $OCH_3$ | | | |
| $C_3H_7$ | H | $OCHF_2$ | | | |

Die Benzodisultame der Formel (II) sind bisher nicht aus der Literatur bekannt. Man erhält die Verbindungen der Formel (II), wenn man Oxyguanidine der allgemeinen Formel (III)

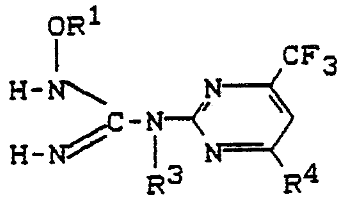

(III)

in welcher
$R^1$, $R^3$ und $R^4$ die oben angegebenen Bedeutungen haben,
mit Benzol-1,2-disulfonsäure-dichlorid, gegebenenfalls in Gegenwart eines Säureakzeptors, wie z. B. Pyridin oder Diazabicyclo[2.2.2]octan (DABCO), und gegebenenfalls in Gegenwart eines Verdünnungsmittels, wie z. B. Methylenchlorid, Chloroform, Tetrahydrofuran oder Dioxan, bei Temperaturen zwischen -30 °C und +50 °C umsetzt.

Die Aufarbeitung kann nach üblichen Methoden erfolgen, beispielsweise durch Einengen, Aufnehmen des Rückstandes in Methylenchlorid, Waschen mit verdünnter Salzsäure und mit Wasser, Abtrennen, Trocknen, Filtrieren und Einengen der organischen Phase, wobei die Produkte der Formel (II) im Rückstand verbleiben.

Das als Ausgangsstoff zu verwendende Benzol-1,2-disulfonsäure-dichlorid ist bereits bekannt (vgl. J. Org. Chem. 31 (1966), 3289 - 3292).

Die weiter als Ausgangsstoffe zu verwendenden Oxyguanidine sind durch die Formel (III) allgemein definiert. In Formel (III) haben $R^1$, $R^3$ und $R^4$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Beispiele für die Ausgangsstoffe der Formel (III) sind in der nachstehenden Tabelle 2 aufgeführt.

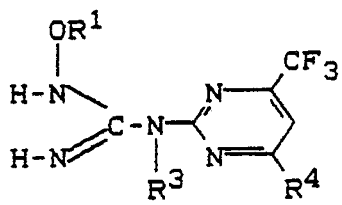

(III)

Tabelle 2: Beispiele für Ausgangsstoffe der Formel (III)

| $R^1$ | $R^3$ | $R^4$ |
|---|---|---|
| $CH_3$ | H | F |
| $CH_3$ | H | Cl |
| $CH_3$ | H | Br |
| $CH_3$ | H | $CH_3$ |
| $CH_3$ | H | $CF_3$ |
| $CH_3$ | H | $OCH_3$ |
| $CH_3$ | H | $OC_2H_5$ |
| $CH_3$ | H | $OCHF_2$ |
| $CH_3$ | H | $NHCH_3$ |
| $CH_3$ | H | $O-C_3H_7-n$ |
| $CH_3$ | H | $O-C_3H_7-i$ |
| $CH_3$ | H | $NHC_2H_5$ |

- Fortsetzung

Tabelle 2: Beispiele für Ausgangsstoffe der Formel (III)

| $R^1$ | $R^3$ | $R^4$ |
|---|---|---|
| $CH_3$ | H | $N(CH_3)_2$ |
| $CH_3$ | $CH_3$ | Cl |
| $CH_3$ | $CH_3$ | $CF_3$ |
| $CH_3$ | $CH_3$ | $CH_3$ |
| $CH_3$ | $CH_3$ | $OCH_3$ |
| $CH_3$ | $CH_3$ | $OC_2H_5$ |
| $CH_3$ | $CH_3$ | $OCHF_2$ |
| $CH_3$ | $CH_3$ | $NHCH_3$ |
| $C_2H_5$ | H | Cl |
| $C_2H_5$ | H | $CF_3$ |
| $C_2H_5$ | H | $OCH_3$ |
| $C_2H_5$ | H | $OC_2H_5$ |
| $C_2H_5$ | H | $OCHF_2$ |
| $C_3H_7$ | H | $OCH_3$ |
| $C_3H_7$ | H | $OCHF_2$ |
| $CH_3$ | H | H |
| $-CH_2-CH=CH_2$ | H | Cl |
| $-CH_2-CH=CH_2$ | H | $CF_3$ |
| $-CH_2-CH=CH_2$ | H | $OCH_3$ |
| $-CH_2-CH=CH_2$ | H | $OC_2H_5$ |
| $-CH_2-CH=CH_2$ | H | $OCHF_2$ |
| $CH(CH_3)_2$ | H | $OCH_3$ |
| $CH(CH_3)_2$ | H | $OCHF_2$ |
| $C_2H_5$ | H | H |

Die Oxyguanidine der Formel (III) sind bisher nicht aus der Literatur bekannt. Man erhält die Verbindungen der Formel (III), wenn man 2-Cyanamino-4-trifluormethyl-pyrimidine der allgemeinen Formel (XIII)

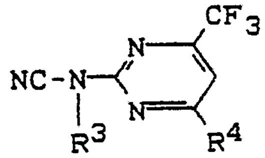

(XIII)

in welcher

$R^3$ und $R^4$ die oben angegebenen Bedeutungen haben,

mit Hydroxylamin-Derivaten der allgemeinen Formel (XIV)

$H_2N - OR^1$ (XIV)

in welcher

$R^1$ die oben angegebene Bedeutung hat,

bzw. mit Hydrochloriden von Hydroxylamin-Derivaten der Formel (XIV) gegebenenfalls in Gegenwart von Verdünnungsmitteln, wie z. B. Ethanol, Propanol oder Butanol, bei Temperaturen zwischen 20 °C und 120 °C umsetzt und gegebenenfalls die Umsetzungsprodukte mit Säureakzeptoren, wie z. B. Ammoniak, Kaliumcarbonat oder Natriumhydroxid in Wasser, behandelt.

Die Hydroxylamin-Derivate der Formel (XIV) sind bekannt und/oder können nach an sich bekannten Verfahren herge stellt werden (vgl. Chem. Pharm. Bull. 15 (1967), 345 -349; Synthesis 1976, 682 - 684).

In Formel (XIV) hat $R^1$ vorzugsweise bzw. insbesondere die gleiche Bedeutung, wie sie oben im Rahmen der Substituentendefinition der Formel (I) vorzugsweise bzw. als insbesondere bevorzugt angegeben ist.

Als Beispiele für die Hydroxylamin-Derivate der Formel (XIV) seien genannt:

O-Methyl-hydroxylamin, O-Ethyl-hydroxylamin, O-Propyl-hydroxylamin, O-Isopropyl-hydroxylamin und O-Allyl-hydroxylamin.

Die weiter als Ausgangsstoffe zu verwendenden 2-Cyanamino-4-trifluormethyl-pyrimidine sind durch die Formel (XIII) allgemein definiert. In Formel (XIII) haben $R^3$ und $R^4$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Beispiele für die 2-Cyanamino-4-trifluormethyl-pyrimidine der Formel (XIII) seien genannt:

2-Cyanamino-6-chlor-4-trifluormethyl-pyrimidin, 2-Cyanamino-6-methoxy-4-trifluormethyl-pyrimidin, 2-Cyanamino-6-ethoxy-4-trifluormethyl-pyrimidin, 2-Cyanamino-6-difluormethoxy-4-trifluormethyl-pyrimidin, 2-Cyanamino-6-methylamino-4-trifluormethyl-pyrimidin, 2-Cyanamino-6-ethylamino-4-trifluormethyl-pyrimidin und 2-Cyanamino-6-dimethylamino-4-trifluormethyl-pyrimidin.

Die 2-Cyanamino-4-trifluormethyl-pyrimidine der Formel (XIII) sind bisher nicht aus der Literatur bekannt. Man erhält die Verbindungen der Formel (XIII) im wesentlichen auf folgenden Synthesewegen:

(a) durch Umsetzung von 2-Methylsulfonyl-4-trifluormethyl-pyrimidinen der allgemeinen Formel (XV)

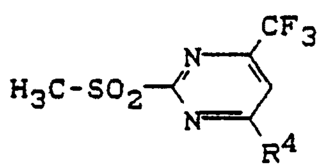

(XV)

in welcher

$R^4$ die oben angegebene Bedeutung hat,

mit Cyanamid-Derivaten der Formel (XVI)

$NC - NH - R^3$ (XVI)

in welcher

$R^3$ die oben angegebene Bedeutung hat,

gegebenenfalls in Gegenwart von Säureakzeptoren, wie z. B. Kaliumcarbonat, und gegebenenfalls in Gegenwart von Verdünnungsmitteln, wie z. B. Acetonitril oder Dimethylformamid, bei Temperaturen zwischen 0 °C und 100 °C. Die Aufarbeitung kann nach üblichen Methoden durchgeführt werden, beispiels weise indem man einengt, den Rückstand in Wasser aufnimmt, mit Essigsäure ansäuert, mit Methylenchlorid extrahiert und aus der Extraktionslösung das Produkt der Formel (XIII) durch Abdestillieren des Lösungsmittels isoliert.

Alternativ erhält man Verbindungen der Formel (XIII)

(b) durch Umsetzung von Cyanoguanidin ("Dicyandiamid") mit geeigneten $\beta$-Dicarbonylverbindungen, wie 1,1,1-Trifluoracetylaceton oder 1,1,1,5,5,5-Hexafluoracetylaceton nach üblichen Methoden (vgl. J. Chem. Soc. 1953, 1725 - 1730).

In einem weiteren Alternativverfahren erhält man Verbindungen der Formel (XIII), wenn man

(c) 2-Amino-4-trifluormethyl-pyrimidine der Formel (XVII)

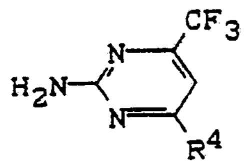

(XVII)

in welcher
$R^4$ die oben angegebene Bedeutung hat,
mit Carbonylisothiocyanaten der Formel (XVIII)

$R^8-\overset{O}{\overset{\|}{C}}-N=C=S$   (XVIII)

in welcher
$R^8$ für Ethoxy oder Phenyl steht,
gegebenenfalls in Gegenwart eines inerten Verdünnungsmittels, wie z. B. Aceton, Acetonitril oder Toluol, bei Temperaturen zwischen 0 °C und 100 °C umsetzt, die hierbei gebildeten Carbonylthioharnstoffe der Formel (XIX)

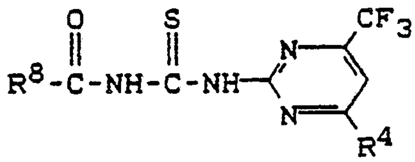

(XIX)

in welcher
$R^4$ und $R^8$ die oben angegebenen Bedeutungen haben,
gegebenenfalls nach Einengen durch Absaugen isoliert und mit wäßrigen Alkalimetall-oder Erdalkalimetallhydroxidlösungen, wie z. B. Natronlauge, gegebenenfalls in Gegenwart eines organischen Lösungsmittels, wie z. B. Tetrahydrofuran oder Dioxan, bei Temperaturen zwischen 0 °C und 120 °C umsetzt und die nach Ansäuren, z. B. mit Salzsäure, kristallin erhaltenen Thioharnstoffe der Formel (XX)

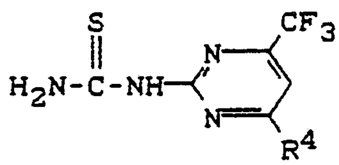

(XX)

in welcher
$R^4$ die oben angegebene Bedeutung hat,
durch Absaugen isoliert und mit Metallverbindungen, welche Schwefelwasserstoff binden können, wie z. B. mit Blei(II)-acetat, Kupfer(II)-acetat, Quecksilber-(II)-acetat oder Eisen(II)-acetat, in Gegenwart von wäßrigen Alkalimetall-oder Erdalkalimetallhydroxid-lösungen, wie z. B. Natronlauge, bei Temperaturen zwischen 20 °C und 100 °C umsetzt, nach Ende der Umsetzung filtriert und das Filtrat mit einer Säure, wie z. B. Essigsäure, ansäuert. Die hierbei kristallin anfallenden Produkte der Formel (XIII) können durch Absaugen isoliert werden.

Die Ausgangsstoffe der Formeln (XVI) und (XVII) sind bekannt und/oder können nach an sich bekannten Verfahren hergestellt werden.

Die Ausgangsstoffe der Formel (XV) sind bisher nicht aus der Literatur bekannt.

Man erhält die 2-Methylsulfonyl-4-trifluormethyl-pyrimidine der Formel (XV), wenn man 2-Methylthio-4-trifluormethyl-pyrimidine der Formel (XXI)

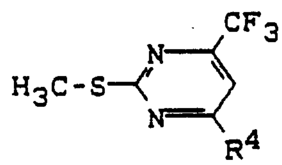

(XXI)

in welcher

$R^4$ die oben angegebene Bedeutung hat,

mit Oxidationsmitteln, wie z. B. Chlorwasser bzw. hypochloriger Säure oder deren Metallsalzlösungen in Wasser, gegebenenfalls in Gegenwart von organischen Lösungsmitteln, wie z. B. Methylenchlorid, Chloroform oder Tetrachlormethan, bei Temperaturen zwischen -20 °C und +30 °C umsetzt. Die Verbindungen der Formel (XV) können durch Abtrennen der organischen Phase und Abdestillieren des Lösungsmittels isoliert werden.

Die als Ausgangsstoffe zu verwendenden 2-Methylthio-4-trifluormethyl-pyrimidine der Formel (XXI) sind bekannt und/oder können nach an sich bekannten Verfahren hergestellt werden (vgl. J. Heterocycl. Chem. 20 (1983), 219 - 223).

Die beim erfindungsgemäßen Verfahren (b) als Ausgangsstoffe zu verwendenden Oxyguanidine sind durch die Formel (III) allgemein definiert. In Formel (III) haben $R^1$, $R^3$ und $R^4$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Beispiele für Verbindungen der Formel (III) wurden bereits weiter oben im Zusammenhang mit der Beschreibung der Ausgangsstoffe für Verfahren (a) genannt. Die Herstellung der Ausgangsstoffe der Formel (III) wurde bereits oben im Zusammenhang mit der Beschreibung der Ausgangsstoffe für Verfahren (a) beschrieben.

Alternativ erhält man die Oxyguanidine der Formel (III) auch, wenn man Thioharnstoffe der Formel (XXa)

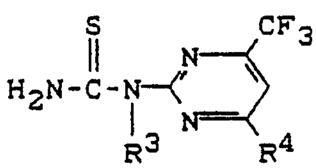

(XXa)

in welcher

$R^3$ und $R^4$ die oben angegebenen Bedeutungen haben,

mit Alkylierungsmitteln, wie z. B. Methyliodid, gegebenenfalls in Gegenwart von Säureakzeptoren, wie z. B. Kaliumcarbonat, und gegebenenfalls in Gegenwart von Verdünnungsmitteln, wie z. B. Aceton, Acetonitril oder Dimethylformamid, nach üblichen Methoden am Schwefelatom alkyliert und die so erhaltenen S-Alkylisothioharnstoffe mit Hydroxylaminen der Formel (XIV)

$H_2N - OR^1$ (XIV

in welcher

$R^1$ die oben angegebene Bedeutung hat,

gegebenenfalls in Gegenwart eines Verdünnungsmittels, wie z. B. Wasser, Methanol, Ethanol oder Dimethylformamid, bei Temperaturen zwischen 0 °C und 100 °C umsetzt (vgl. Houben-Weyl, Methoden der organischen Chemie, Band 8, S. 184 und Band E4, S. 614).

Das beim erfindungsgemäßen Verfahren (b) weiter als Ausgangsstoff zu verwendende Benzol-1,2-disulfonsäure-dichlorid ist bereits bekannt (vgl. J. Org. Chem. 31 (1966), 3289 - 3292).

Die beim erfindungsgemäßen Verfahren (c) als Ausgangsstoffe zu verwendenden 2-Amino-4-trifluormethyl-pyrimidine sind durch die Formel (IV) allgemein definiert. In Formel (IV) haben $R^3$ und $R^4$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Beispiele für die Ausgangsstoffe der Formel (IV) seien genannt:

2-Amino-4-trifluormethyl-6-methoxy-pyrimidin, 2-Amino-4-trifluormethyl-6-ethoxy-pyrimidin, 2-Amino-4-trifluormethyl-6-chlor-pyrimidin, 2-Amino-4-trifluormethyl-6-difluormethoxy-pyrimidin, 2-Methylamino-4-trifluormethyl-6-methoxy-pyrimidin und 2-Methylamino-4-trifluormethyl-6-difluormethoxy-pyrimidin.

Die 2-Amino-4-trifluormethyl-pyrimidine der Formel (IV) sind bekannt und/oder können nach an sich bekannten Verfahren hergestellt werden (vgl. JP 12903/67 oder Chem. Abstracts 68 (1968), 12988v; J. Org. Chem. 49 (1984), 4072 - 4074).

Die weiter beim erfindungsgemäßen Verfahren (c) als Ausgangsstoffe zu verwendenden 2-Oxyaminosulfonyl-phenylsulfonyl-iso(thio)cyanate sind durch die Formel (V) allgemein definiert. In Formel (V) haben $R^1$ und $R^2$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Beispiele für die Ausgangsstoffe der Formel (V) seien genannt:

2-(N-Methoxy-N-methyl-aminosulfonyl)-, 2-(N-Ethoxy-N-methyl-aminosulfonyl)-, 2-(N-Allyloxy-N-methyl-aminosulfonyl)-, 2-(N-Methoxy-N-ethyl-aminosulfonyl)-, 2-(N-Ethoxy-N-ethyl-aminosulfonyl)-, 2-(N-Allyloxy-N-ethyl-aminosulfonyl)-, 2-(N-Methoxy-N-allyl-aminosulfonyl)-, 2-(N-Ethoxy-N-allyl-aminosulfonyl)-und 2-(N-Allyloxy-N-allyl-aminosulfonyl)-phenylsulfonyl-isocyanat bzw. -phenylsulfonyl-isothiocyanat.

Die 2-Oxyaminosulfonyl-phenylsulfonyl-iso(thio)cyanate der Formel (V) sind bekannt und/oder können nach an sich bekannten Verfahren hergestellt werden (vgl. EP-A 70 041).

Man erhält diese Verbindungen, wenn man

(a) für den Fall, daß Q für Sauerstoff steht, 2-Oxyaminosulfonyl-benzolsulfonsäureamide der Formel (VIII)

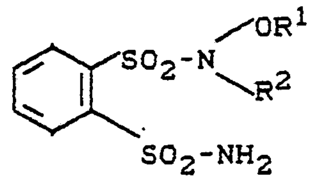

(VIII)

in welcher

$R^1$ und $R^2$ die oben angegebenen Bedeutungen haben,

mit etwa der äquimolaren Menge eines Alkylisocyanats, wie z. B. Butylisocyanat, gegebenenfalls in Gegenwart eines Katalysators, wie z. B. Diazabicyclo[2.2.2]octan (DABCO) und in Gegenwart eines Verdünnungsmittels, wie z. B. Xylol, bei Temperaturen zwischen 50 °C und 200 °C, vorzugsweise zwischen 80 °C und 150 °C, umsetzt und dabei mindestens die äquimolare Menge Phosgen einleitet, oder

(b) für den Fall, daß Q für Schwefel steht, wenn man 2-Oxyaminosulfonyl-benzolsulfonsäureamide der Formel (VIII) - oben - mit Schwefelkohlenstoff in Gegenwart eines Säureakzeptors, wie z. B. Kaliumhydroxid, und gegebenenfalls in Gegenwart eines Verdünnungsmittels, wie z. B. Dimethylformamid, bei Temperaturen zwischen 0 °C und 50 °C umsetzt und anschließend mit Phosgen oder Thionylchlorid, gegebenenfalls in Gegenwart eines Verdünnungsmittels, wie z. B. Methylenchlorid, bei Temperaturen zwischen 0 °C und 50 °C umsetzt (vgl. Arch. Pharm. 299 (1966), 174).

Die beim erfindungsgemäßen Verfahren (d) als Ausgangsstoffe zu verwendenden 1-(2-Oxyaminosulfonyl-phenylsulfonyl)-3-(4-trifluormethyl-pyrimidin-2-yl)-(thio)harnstoffe sind durch die Formel (VI) allgemein definiert. In Formel (VI) haben Q, $R^1$, $R^3$ und $R^4$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Beispiele für die Ausgangsstoffe der Formel (VI) sind in der nachstehenden Tabelle 3 aufgeführt.

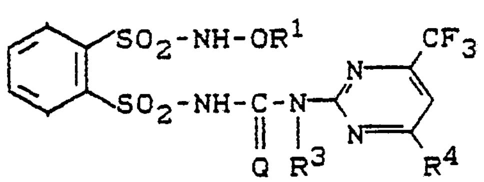

(VI)

Tabelle 3: Beispiele für Ausgangsstoffe der Formel (VI)

| Q | $R^1$ | $R^3$ | $R^4$ |
|---|---|---|---|
| O | $CH_3$ | H | F |
| O | $CH_3$ | H | Cl |
| O | $CH_3$ | H | Br |
| O | $CH_3$ | H | $CH_3$ |
| O | $CH_3$ | H | $CF_3$ |
| O | $CH_3$ | H | $OCH_3$ |

Tabelle 3 - Fortsetzung

| Q | $R^1$ | $R^3$ | $R^4$ |
|---|---|---|---|
| O | $CH_3$ | H | $OC_2H_5$ |
| O | $CH_3$ | H | $OC_3H_7$-n |
| O | $CH_3$ | H | $OC_3H_7$-i |
| O | $CH_3$ | H | $OCHF_2$ |
| O | $CH_3$ | H | $NHCH_3$ |
| O | $CH_3$ | H | $NHC_2H_5$ |
| O | $CH_3$ | H | $N(CH_3)_2$ |
| O | $CH_3$ | $CH_3$ | Cl |
| O | $CH_3$ | $CH_3$ | $CF_3$ |
| O | $CH_3$ | $CH_3$ | $CH_3$ |
| O | $CH_3$ | $CH_3$ | $OCH_3$ |
| O | $CH_3$ | $CH_3$ | $OC_2H_5$ |
| O | $CH_3$ | $CH_3$ | $OCHF_2$ |
| O | $CH_3$ | $CH_3$ | $NHCH_3$ |
| O | $C_2H_5$ | H | Cl |
| O | $C_2H_5$ | H | $CF_3$ |
| O | $C_2H_5$ | H | $OCH_3$ |
| O | $C_2H_5$ | H | $OC_2H_5$ |
| O | $C_2H_5$ | H | $OCHF_2$ |
| O | $C_3H_7$ | H | $OCH_3$ |
| O | $C_3H_7$ | H | $OCHF_2$ |

Tabelle 3 - Fortsetzung

| Q | $R^1$ | $R^3$ | $R^4$ |
|---|---|---|---|
| O | $-CH_2-CH=CH_2$ | H | Cl |
| O | $-CH_2-CH=CH_2$ | H | $CF_3$ |
| O | $-CH_2-CH=CH_2$ | H | $OCH_3$ |
| O | $-CH_2-CH=CH_2$ | H | $OC_2H_5$ |
| O | $-CH_2-CH=CH_2$ | H | $OCHF_2$ |
| O | $CH(CH_3)_2$ | H | $OCH_3$ |
| O | $CH(CH_3)_2$ | H | $OCHF_2$ |
| S | $CH_3$ | H | $CH_3$ |
| S | $CH_3$ | H | $CF_3$ |
| S | $CH_3$ | H | $OCH_3$ |
| S | $CH_3$ | H | $OC_2H_5$ |
| S | $C_2H_5$ | H | $OCH_3$ |
| S | $CH_3$ | H | $OCHF_2$ |
| S | $C_2H_5$ | H | $OCHF_2$ |
| S | $CH_3$ | $CH_3$ | $OCH_3$ |
| O | $CH_3$ | H | H |
| O | $C_2H_5$ | H | H |
| O | $-CH_2-CH=CH_2$ | H | H |

Die bei Verfahren (d) als Ausgangsstoffe zu verwendenden 1-(2-Oxyaminosufonyl-phenylsulfonyl)-3-(4-trifluormethyl-pyrimidin-2-yl)-(thio)harnstoffe der Formel (VI) werden durch die allgemeine Formel (I) umfasst und können nach den erfindungsgemäßen Verfahrensvarianten (a) und (b) hergestellt werden.

Die bei Verfahren (d) weiter als Ausgangsstoffe zu verwendenden Verbindungen sind durch die Formel (VII) allgemein definiert. In Formel (VII) hat $R^2$ vorzugsweise bzw. insbesondere die gleiche Bedeutung, wie sie oben im Rahmen der Substituentendefinition der Formel (I) vorzugsweise bzw. als insbesondere bevorzugt angegeben ist und X steht vorzugsweise wie auch insbesondere für Chlor oder Brom.

Als Beispiele für die Ausgangsstoffe der Formel (VII) seien genannt:
Methylchlorid, Methylbromid, Ethylbromid, 1-Brom-propan, 2-Brom-propan, Allylchlorid, Allylbromid, Crotylchlorid, Propargylchlorid, Acetylchlorid, Propionsäurechlorid, Buttersäurechlorid, Chloracetylchlorid, Dichloracetylchlorid, Trichloracetylchlorid, Cyclopropancarbonsäurechlorid, Cyclobutancarbonsäurechlorid, Cyclopentancarbonsäurechlorid, Cyclohexancarbonsäurechlorid, Acrylsäurechlorid, Crotonsäurechlorid, Phenylessigsäurechlorid, Benzoesäurechlorid, 4-Fluorbenzoesäurechlorid, 4-Chlor-benzoesäurechlorid, 2-Fluor-benzoesäurechlorid, 3-Fluor-benzoesäurechlorid, 4-Nitro-benzoesäurechlorid, 4-Methyl-benzoesäurechlorid, 4-Trifluormethyl-benzoesäurechlorid, 4-Methoxy-benzoesäurechlorid, 3-Pyridincarbonsäurechlorid und 2-Furan-carbonsäurechlorid. Die Ausgangsstoffe der Formel (VII) sind bekannte organische Chemikalien.

Das erfindungsgemäße Verfahren (a) zur Herstellung der neuen Verbindungen der Formel (I) wird vorzugsweise unter Verwendung eines Verdünnungsmittels durchgeführt. Als Verdünnungsmittel kommen alle inerten organischen Lösungsmittel, vorzugsweise jedoch aprotisch polare Solventien in Betracht, z. B. Methylenchlorid, Tetrahydrofuran, Dioxan, Aceton, Methylethylketon, Methylisopropylketon, Methylisobutylketon, Acetonitril, Propionitril, Dimethylformamid und Dimethylsulfoxid. Im Falle der Umsetzung mit Wasser kann dieses auch als Verdünnungsmittel dienen.

Verfahren (a) wird gegebenenfalls - bei der Umsetzung mit Wasser vorzugsweise - in Gegenwart eines Katalysators durchgeführt. Als Katalysatoren kommen Säuren, wie z. B. Salzsäure oder Schwefelsäure, oder Basen, wie z. B. Natronlauge oder Kalilauge, in Betracht.

Die Reaktionstemperaturen können bei dem erfindungsgemäßen Verfahren (a) in einem größeren Bereich variiert werden. Im allgemeinen arbeitet man bei Temperaturen zwischen 0 °C und 100 °C, vorzugsweise bei Temperaturen zwischen 10 °C und 80 °C. Verfahren (a) wird im allgemeinen bei Normaldruck durchgeführt.

Zur Durchführung von Verfahren (a) setzt man je Mol Benzodisultam der Formel (II) im allgemeinen zwischen 1 und 100 Mol, vorzugsweise zwischen 5 und 50 Mol Wasser bzw. zwischen 1 und 10 Mol, vorzugsweise zwischen 1,5 und 5 Mol, Schwefelwasserstoff ein.

Im Falle der Umsetzung mit Wasser werden die Reaktionskomponenten gewöhnlich bei Raumtemperatur zusammengegeben und das Reaktionsgemisch wird bis zum Reaktionsende gerührt.

Im Falle der Umsetzung mit Schwefelwasserstoff wird das Benzodisultam der Formel (II) vorzugsweise in einem geeigneten Verdünnungsmittel vorgelegt und unter Rühren wird Schwefelwasserstoff bis zum Reaktionsende eingeleitet.

Die Aufarbeitung kann auf übliche Weise durchgeführt werden, beispielsweise indem man gegebenenfalls mit Salzsäure ansäuert, gegebenenfalls auf etwa die Hälfte einengt und das kristallin angefallene Produkt der Formel (I) durch Absaugen isoliert.

Das erfindungsgemäße Verfahren (b) zur Herstellung der neuen Verbindungen der Formel (I) wird vorzugsweise unter Verwendung von Verdünnungsmitteln durchgeführt. Als solche kommen praktisch alle inerten organischen, vorzugsweise aprotisch polare Solventien in Betracht. Hierzu gehören gegebenenfalls substituierte Kohlenwasserstoffe, wie z. B. Methylenchlorid, Chloroform, 1,2-Dichlorethan, Toluol, Xylol und Chlorbenzol, Nitrile, wie z. B. Acetonitril und Propionitril, Ether, wie z. B. 1,2-Dimethoxyethan, Tetrahydrofuran und Dioxan, sowie Dimethylformamid, Dimethylacetamid, Dimethylsulfoxid, Sulfolan, Pyridin und 2-Methyl-5-ethyl-pyridin.

Als Säureakzeptoren können bei Verfahren (b) praktisch alle üblicherweise verwendeten Säurebindemittel eingesetzt werden. Hierzu gehören insbesondere Alkalimetall-und Erdalkalimetallhydroxide, Alkalimetall-und Erdalkalimetallhydride, metallorganische Verbindungen, wie Butyllithium, ferner aliphatische, aromatische oder heterocyclische Amine, wie Trimethylamin, Triethylamin, N,N-Dimethyl-anilin, N,N-Dimethyl-benzylamin, Diazabicyclooctan (DABCO), Diazabicyclononen (DBN), Diazabicycloundecen (DBU), Pyridin, 2-Methyl-5-ethyl-pyridin und 4-Dimethylaminopyridin.

Bei Verfahren (b) können die gleichen Katalysatoren wie bei Verfahren (a) verwendet werden.

Die Reaktionstemperaturen können bei Verfahren (b) innerhalb eines größeren Bereiches variiert werden. Im allgemeinen arbeitet man zwischen -80 °C und + 100 °C, vorzugsweise zwischen -30 °C und +50 °C. Das erfindungsgemäße Verfahren (b) wird im allgemeinen bei Normaldruck durchgeführt.

Zur Durchführung von Verfahren (b) setzt man je Mol Oxyguanidin-Derivat der Formel (III) im allgemeinen zwischen 1 und 2 Mol, vorzugsweise zwischen 1,0 und 1,2 Mol Benzol-1,2-disulfonsäure-dichlorid und anschließend zwischen 1 und 100 Mol, vorzugsweise zwischen 5 und 50 Mol Wasser bzw. zwischen 1 und 10 Mol, vorzugsweise zwischen 1,5 und 5 Mol, Schwefelwasserstoff ein.

Die Reaktionskomponenten werden gewöhnlich bei Raumtemperatur oder unter Außenkühlung zusammengegeben und das Reaktionsgemisch wird bis zum Reaktionsende gerührt.

Die Aufarbeitung kann auf gleiche Weise wie bei Verfahren (a) durchgeführt werden.

Das erfindungsgemäße Verfahren (c) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan. Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl-und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren (c) wird gegebenenfalls in Gegenwart eines Katalysators durchgeführt. Hierzu gehören insbesondere aliphatische, aromatische oder heterocyclische Amine, wie Triethylamin, N,N-Dimethylanilin, Pyridin, 2-Methyl-5-ethyl-pyridin, 4-Dimethylamino-pyridin, Diazabicyclooctan (DABCO), Diazabicyclononen (DBN) und Diazabicycloundecen (DBU).

Die Reaktionstemperaturen können bei dem erfindungsgemäßen Verfahren (c) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) setzt man je Mol 2-Amino-4-trifluormethyl-pyrimidin der Formel (IV) im allgemeinen 1,0 bis 3,0 Mol, vorzugsweise 1,0 bis 2,0 Mol 2-Oxyaminosulfonylphenylsulfonyl-iso(thio)cyanat der Formel (V) ein.

Die Ausgangsstoffe der Formeln (IV) und (V) und gegebenenfalls der Katalysator und das Verdünnungsmittel werden im allgemeinen bei Raumtemperatur oder unter leichter Aussenkühlung zusammengegeben und das Reaktionsgemisch wird gegebenenfalls bei erhöhter Temperatur bis zum Reaktionsende gerührt.

Die Aufarbeitung und Isolierung der neuen Verbindungen der Formel (I) erfolgt nach üblichen Methoden:

Soweit die Verbindungen der Formel (I) kristallin anfallen, werden sie durch Absaugen isoliert. Andernfalls wird - gegebenenfalls nach Einengen - Wasser und ein mit Wasser praktisch nicht mischbares organisches Lösungsmittel dazu gegeben, nach Durchschütteln die organische Phase abgetrennt, getrocknet, filtriert und eingeengt, wobei die Produkte der Formel (I) im Rückstand verbleiben.

Das erfindungsgemäße Verfahren (d) zur Herstellung der neuen Verbindungen der Formel (I) wird vorzugsweise unter Verwendung von Verdünnungsmitteln durchgeführt. Hierbei kommen die gleichen Verdünnungsmittel in Betracht, wie sie oben für Verfahren (c) angegeben sind.

Verfahren (d) wird gegebenenfalls in Gegenwart eines Säureakzeptors durchgeführt. Hierbei kommen die gleichen Säureakzeptoren in Betracht, wie sie oben für Verfahren (b) angegeben sind.

Die Reaktionstemperaturen können bei dem erfindungsgemäßen Verfahren (d) in einem größeren Bereich variiert werden. Im allgemeinen arbeitet man bei Temperaturen zwischen -20 °C und +60 °C, vorzugsweise bei Temperaturen zwischen 0 °C und +40 °C. Verfahren (d) wird im allgemeinen bei Normaldruck durchgeführt.

Zur Durchführung von Verfahren (d) setzt man auf 1 Mol der Verbindung der Formel (VI) im allgemeinen 1 bis 10 Mol, vorzugsweise 2 bis 8 Mol einer Verbindung der Formel (VII) ein.

Die Reaktionskomponenten werden gewöhnlich bei Raumtemperatur oder unter Außenkühlung zusammengegeben und das Reaktionsgemisch wird bis zum Reaktionsende gerührt.

Die Aufarbeitung kann auf übliche Weise durchgeführt werden; beispielsweise indem man gegebenenfalls einengt und/oder mit einem mit Wasser praktisch nicht mischbaren organischen Lösungsmittel, wie z. B. Methylenchlorid, verdünnt, mit Wasser wäscht, trocknet, filtriert und einengt. Das kristallin anfallende Produkt der Formel (I) kann durch Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die erfindungsgemäßen Verbindungen eignen sich vor allem zur selektiven Bekämpfung dikotyler Unkräuter in monokotylen Kulturen im Vorauflauf-und im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur-und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

In Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder Schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage; ferner auch Bentazon, Bifenox, Bromoxynil, Chlorsulfuron, Chlortoluron, Fluroxypyr, Illoxan, Imazamethabenz, loxynil, Isoproturon, MCPA, MCPP, Mefenacet, Pendimethalin, Sulfometuron, Terbutryne, Whip, Amethydione, 2,4-D, 2,4-DP, [(4-Amino-3,5-dichlor-6-fluor-2-pyridinyl)-oxy]-essigsäure und deren 1-Methylheptylester und 2-[4-(3,5-Dichlor-2-pyridyl-oxy)-phenoxy]-propionsäure-(trimethylsilylmethylester). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres

Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

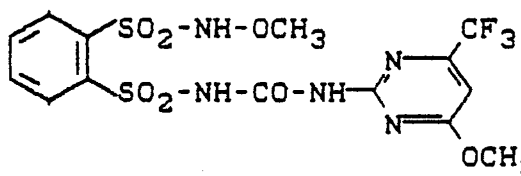

(Verfahrensvariante (b))

5,6 g (0,02 Mol) Benzol-1,2-disulfonsäure-dichlorid werden portionsweise bei -20 °C zu einer Mischung aus 5,3 g (0,02 Mol) N'-(4-Trifluormethyl-6-methoxy-pyrimidin-2-yl)-N"-methoxy-guanidin, 2,3 g (0,02 Mol) Diazabicyclo-[2.2.2]octan und 60 ml Tetrahydrofuran unter Rühren gegeben. Das Reaktionsgemisch wird dann 15 Stunden bei +20 °C gerührt, anschließend mit 2N-Natronlauge auf einen pH-Wert von 12 bis 14 gebracht und 3 Stunden bei 20 °C gerührt. Anschließend wird der pH-Wert durch Zugabe von konz. Salzsäure auf 1 eingestellt und nach Kühlen mit Eis wird das kristallin angefallene Produkt durch Absaugen isoliert.

Man erhält 4,6 g (47 % der Theorie) 1-(2-Methoxyaminosulfonyl-phenylsulfonyl)-3-(4-trifluormethyl-6-methoxy-pyrimidin-2-yl)-harnstoff vom Schmelzpunkt 217 °C.

Beispiel 2

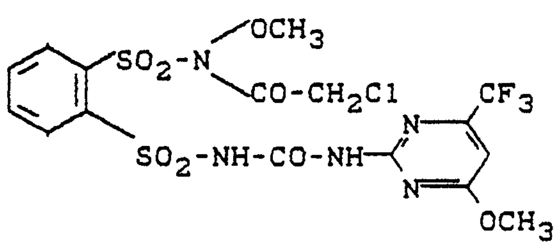

(Verfahrensvariante (d))

5,3 g (0,047 Mol) Chloracetylchlorid werden bei 20 °C bis 30 °C portionsweise zu einer Mischung aus 3,4 g (0,007 Mol) 1-(2-Methoxyaminosulfonyl-phenylsulfonyl)-3-(4-trifluormethyl-6-methoxy-pyrimidin-2-yl)-harnstoff - Herstellung vgl. Beispiel 1 - und 4,4 g (0,036 Mol) 4-Dimethylamino-pyridin in 50 ml Methylenchlorid unter Rühren gegeben. Das Reaktionsgemisch wird dann 15 Stunden bei 20 °C gerührt; anschließend

wird durch Zugabe von 2N-Salzsäure ein pH-Wert zwischen 2 und 5 eingestellt, die organische Phase abgetrennt, mit Wasser gewaschen, mit Natriumsulfat getrocknet und filtriert. Das Filtrat wird eingeengt, der Rückstand mit Diethylether digeriert und das hierbei kristallin angefallene Produkt durch Absaugen isoliert.

Man erhält 2,9 g (74 % der Theorie) 1-(2-(N-Chloracetyl-N-methoxy-amino)-sulfonyl-phenylsulfonyl)-3-(4-trifluormethyl-6-methoxy-pyrimidin-2-yl)-harnstoff vom Schmelzpunkt 197 °C.

Beispiel 3

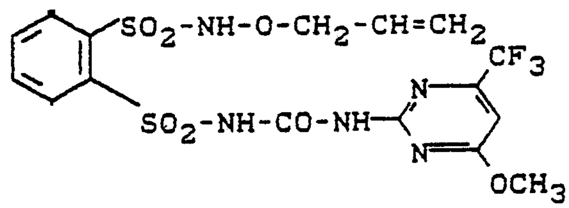

(Verfahrensvariante (a))

8,0 g (0,016 Mol) Benzodisultam der Formel (II-16) ($R^1$ = $CH_2$-$CH$=$CH_2$, $R^3$ = H, $R^4$ = $OCH_3$) werden in einer Mischung aus 50 ml Tetrahydrofuran, 40 ml Wasser und 10 ml konz. Salzsäure 7 Tage bei 20 °C gerührt, bis laut DC-Kontrolle die Umsetzung beendet ist. Der Reaktionsansatz wird im Vakuum eingeengt, in Methylenchlorid aufgenommen, neutral gewaschen, getrocknet und eingeengt. Der Rückstand wird mit Ether kristallin verrieben.

Man erhält 3,5 g (0,007 Mol, 42 % der Theorie) 1-(2-Allyloxyaminosulfonyl-phenylsulfonyl)-3-(4-trifluormethyl-6-methoxy-pyrimidin-2-yl)-harnstoff vom Schmelzpunkt 173 °C (Zers.).

Analog Beispiel 1 und 2 und entsprechend der allgemeinen Beschreibung der erfindungsgemäßen Herstellungsverfahren können die in der nachstehenden Tabelle 4 aufgeführten Verbindungen der allgemeinen Formel (I) hergestellt werden.

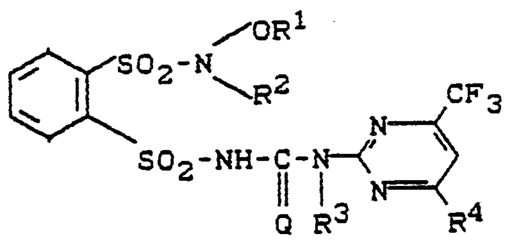

(I)

Tabelle 4: Beispiele für Verbindungen der Formel (I)

| Bsp.-Nr. | Q | $R^1$ | $R^2$ | $R^3$ | $R^4$ | Schmelzpunkt/°C |
|---|---|---|---|---|---|---|
| 4 | O | $C_2H_5$ | H | H | $OCH_3$ | |
| 5 | O | $CH_3$ | H | H | Cl | |
| 6 | O | $CH_3$ | $-CO-CH_2-$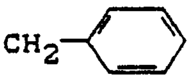 | H | $OCH_3$ | 172 |
| 7 | S | $CH_3$ | H | H | $OCH_3$ | 193 |
| 8 | O | $CH_3$ | H | H | $OCHF_2$ | |
| 9 | O | $CH_3$ | H | $CH_3$ | $CH_3$ | |
| 10 | S | $CH_3$ | H | H | $CH_3$ | |
| 11 | O | $CH_3$ | $-CO-CH_3$ | H | $OCH_3$ | 196 |
| 12 | S | $CH_3$ | H | H | $CF_3$ | |
| 13 | S | $C_2H_5$ | H | H | $OCH_3$ | |
| 14 | O | $-CH_2-CH=CH_2$ | H | H | $OCHF_2$ | |
| 15 | O | $C_3H_7$ | $-CO-CH_3$ | H | $OCH_3$ | |
| 16 | O | $CH(CH_3)_2$ | H | H | $OCH_3$ | |
| 17 | O | $CH_3$ | H | H | $OC_2H_5$ | 191 |
| 18 | O | $CH_3$ | -CO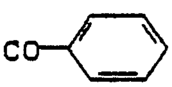 | H | $OCH_3$ | 157 |
| 19 | S | $CH_3$ | H | H | $CH_3$ | |
| 20 | O | $CH_3$ | H | H | $CH_3$ | |
| 21 | O | $CH_3$ | H | H | $CF_3$ | |
| 22 | O | $CH_3$ | H | H | $NHCH_3$ | |

Tabelle 4 - Fortsetzung

| Bsp.-Nr. | Q | $R^1$ | $R^2$ | $R^3$ | $R^4$ | Schmelzpunkt/° C |
|---|---|---|---|---|---|---|
| 23 | O | $CH_3$ | H | H | $NHC_2H_5$ | |
| 24 | O | $CH_3$ | H | H | $N(CH_3)_2$ | 223 |
| 25 | O | $CH_3$ | 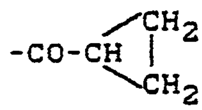 | H | $OCH_3$ | 178 |
| 26 | O | $CH_3$ | H | $CH_3$ | $OCH_3$ | |
| 27 | O | $C_2H_5$ | H | H | Cl | |
| 28 | O | $C_2H_5$ | H | H | $OCHF_2$ | |
| 29 | O | $-CH_2-CH=CH_2$ | H | H | $CF_3$ | |
| 30 | O | $CH_3$ | H | $CH_3$ | Cl | |
| 31 | O | $CH_3$ | H | $CH_3$ | $CF_3$ | |
| 32 | O | $CH_3$ | H | $CH_3$ | $OCHF_2$ | |
| 33 | O | $CH_3$ | H | $CH_3$ | $OC_2H_5$ | |
| 34 | O | $C_2H_5$ | H | H | $CF_3$ | |
| 35 | O | $CH_3$ | $CH_3$ | H | Cl | |
| 36 | O | $CH_3$ | $CH_3$ | H | $OCH_3$ | |
| 37 | S | $CH_3$ | $CH_3$ | H | $OCH_3$ | |
| 38 | O | $CH_3$ | $CH_3$ | H | $OCHF_2$ | |
| 39 | O | $-CH_2-CH_2-CH_2-$ | | H | Cl | |
| 40 | O | $-CH_2-CH_2-CH_2-$ | | H | $OCH_3$ | |
| 41 | O | $-CH_2-CH_2-CH_2-$ | | H | $OCHF_2$ | |
| 42 | O | $-CH_2-CH=CH_2$ | $CH_3$ | H | Cl | |

Tabelle 4 - Fortsetzung

| Bsp.-Nr. | Q | $R^1$ | $R^2$ | $R^3$ | $R^4$ | Schmelz-punkt/°C |
|---|---|---|---|---|---|---|
| 43 | O | $CH_3$ | $-CH_2-CH=CH_2$ | H | Cl | |
| 44 | O | $-CH_2-CH=CH_2$ | $CH_3$ | H | $OCH_3$ | |
| 45 | O | $CH_3$ | $-CH_2-CH=CH_2$ | H | $OCH_3$ | |
| 46 | O | $-CH_2-CH=CH_2$ | $CH_3$ | H | $OCHF_2$ | |
| 47 | O | $CH_3$ | $-CH_2-CH=CH_2$ | H | $OCHF_2$ | |
| 48 | O | $-CH_2-CH=CH_2$ | H | H | Cl | |
| 49 | O | $CH_3$ | $-CO-C_2H_5$ | H | $OCH_3$ | |
| 50 | O | $CH_3$ | $-CO-CHCl_2$ | H | $OCH_3$ | |
| 51 | O | $CH_3$ | -CO 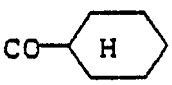 | H | $OCH_3$ | |
| 52 | O | $CH_3$ | -CO 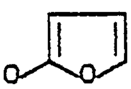 | H | $OCH_3$ | |
| 53 | O | $CH_3$ | -CO 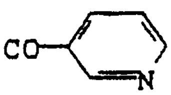 | H | $OCH_3$ | |
| 54 | O | $CH_3$ | -CO 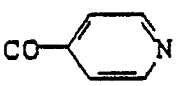 | H | $OCH_3$ | |
| 55 | O | $CH_3$ | -CO 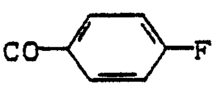 | H | $OCH_3$ | |
| 56 | O | $CH_3$ | H | Na | $OCH_3$ | 152 |
| 57 | O | $CH_3$ | H | $H_2NCH(CH_3)_2$ | $OCH_3$ | 118 |
| 58 | O | $-CH_2-CH=CH_2$ | H | K | $OCH_3$ | |

Tabelle 4 - Fortsetzung

| Bsp.-Nr. | Q | $R^1$ | $R^2$ | $R^3$ | $R^4$ | Schmelzpunkt/°C |
|---|---|---|---|---|---|---|
| 59 | O | $CH_3$ | H | K | $OCH_3$ | |
| 60 | O | $CH_3$ | H | $H_2N(C_4H_9)_2$ | $OCH_3$ | |
| 61 | O | $CH_3$ | H | $H_2NCH(CH_3)_2$ | $OC_2H_5$ | 109 |
| 62 | O | $CH_3$ | H | $H_2NCH(CH_3)_2$ | $N(CH_3)_2$ | 157 |

Ausgangsstoffe der Formel (II)

Beispiel (II-1)

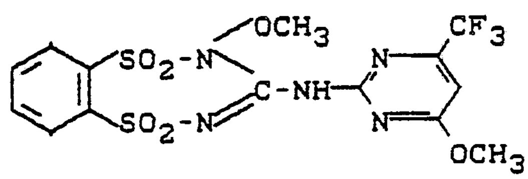

5,6 g (0,02 Mol) Benzol-1,2-disulfonsäure-dichlorid werden portionsweise bei -20 °C zu einer Mischung aus 5,3 g (0,02 Mol) N'-(4-Trifluormethyl-6-methoxy-pyrimidin-2-yl)-N"-methoxy-guanidin, 2,3 g (0,02 Mol) Diazabicyclo[2.2.2]octan und 100 ml Methylenchlorid unter Rühren gegeben. Das Reaktionsgemisch wird dann 15 Stunden bei +20 °C gerührt. Nach Filtration wird das Filtrat eingeengt, der Rückstand mit Diethylether digeriert und das hierbei kristallin angefallene Produkt durch Absaugen isoliert.

Man erhält 8,6 g (92 % der Theorie) der Verbindung der oben angegebenen Strukturformel vom Schmelzpunkt 184 °C.

Analog Beispiel (II-1) können die in der nachstehenden Tabelle 5 aufgeführten Verbindungen der Formel (II) (vgl. auch Tabelle 1) hergestellt werden.

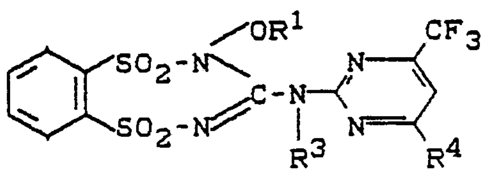

(II)

Tabelle 5: Beispiele für Ausgangsstoffe der Formel (II)

| Beisp. Nr. | $R^1$ | $R^3$ | $R^4$ | Schmelzpunkt [°C] |
|---|---|---|---|---|
| (II-2) | $CH_3$ | H | $OC_2H_5$ | 167 |
| (II-3) | $CH_3$ | H | Cl | |
| (II-4) | $CH_3$ | H | $CH_3$ | |
| (II-5) | $CH_3$ | H | $CF_3$ | |
| (II-6) | $CH_3$ | H | $OCHF_2$ | |
| (II-7) | $C_2H_5$ | H | Cl | |
| (II-8) | $C_2H_5$ | H | $OCH_3$ | |
| (II-9) | $C_2H_5$ | H | $OC_2H_5$ | |
| (II-10) | $C_2H_5$ | H | $CH_3$ | |
| (II-11) | $C_2H_5$ | H | $CF_3$ | |
| (II-12) | $C_2H_5$ | H | $OCF_2H$ | |
| (II-13) | $C_3H_7$ | H | Cl | |
| (II-14) | $C_3H_7$ | H | $OCHF_2$ | |
| (II-15) | $-CH_2-CH=CH_2$ | H | Cl | |
| (II-16) | $-CH_2-CH=CH_2$ | H | $OCH_3$ | 82 |
| (II-17) | $-CH_2-CH=CH_2$ | H | $OCHF_2$ | |
| (II-18) | $-CH_2-CH=CH_2$ | H | $CF_3$ | |
| (II-19) | $CH_3$ | H | $O-C_3H_7-n$ | |
| (II-20) | $CH_3$ | H | $O-C_3H_7-i$ | |
| (II-21) | $CH_3$ | H | $N(CH_3)_2$ | |

Ausgangsstoffe der Formel (III)

Beispiel (III-1)

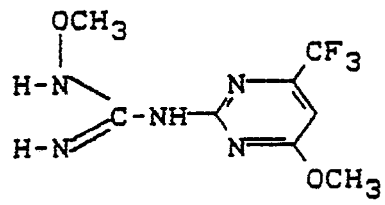

Eine Mischung aus 15,5 g (0,07 Mol) 2-Cyanamino-4-trifluormethyl-6-methoxy-pyrimidin, 9,4 g (0,1 Mol) O-Methyl-hydroxylamin-Hydrochlorid und 100 ml Ethanol wird 48 Stunden unter Rückfluß zum Sieden erhitzt, heiß filtriert, auf etwa das halbe Volumen eingeengt, mit Wasser auf etwa die dreifache Menge verdünnt, mit 2N-Natronlauge auf einen pH-Wert von 9 eingestellt und mit Methylenchlorid extrahiert. Die organische Phase wird mit Natriumsulfat getrocknet, filtriert und das Filtrat eingeengt, wobei das Produkt als kristalliner Rückstand verbleibt.

Man erhält 10,8 g (58 % der Theorie) N'-(4-Trifluormethyl-6-methoxy-pyrimidin-2-yl)-N''-methoxy-guanidin vom Schmelzpunkt 101 °C.

Analog Beispiel (III-1) können die in der nachstehenden Tabelle 6 aufgeführten Verbindungen der Formel (III) (vgl. auch Tabelle 2) hergestellt werden.

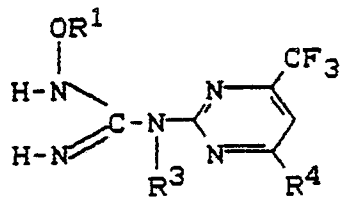

(III)

Tabelle 6: Beispiele für Ausgangsstoffe der Formel (III)

| Beisp. Nr. | $R^1$ | $R^3$ | $R^4$ | Schmelzpunkt [°C] |
|---|---|---|---|---|
| (III-2) | $CH_3$ | H | $OC_2H_5$ | 103 |
| (III-3) | $CH_3$ | H | Cl | |
| (III-4) | $CH_3$ | H | $CH_3$ | |
| (III-5) | $CH_3$ | H | $CF_3$ | |
| (III-6) | $CH_3$ | H | $OCHF_2$ | |
| (III-7) | $C_2H_5$ | H | Cl | |
| (III-8) | $C_2H_5$ | H | $OCH_3$ | |
| (III-9) | $C_2H_5$ | H | $OC_2H_5$ | |
| (III-10) | $C_2H_5$ | H | $CH_3$ | |
| (III-11) | $C_2H_5$ | H | $CF_3$ | |
| (III-12) | $C_2H_5$ | H | $OCF_2H$ | |
| (III-13) | $C_3H_7$ | H | Cl | |
| (III-14) | $C_3H_7$ | H | $OCHF_2$ | |
| (III-15) | $-CH_2-CH=CH_2$ | H | Cl | |
| (III-16) | $-CH_2-CH=CH_2$ | H | $OCH_3$ | 82 |
| (III-17) | $-CH_2-CH=CH_2$ | H | $OCHF_2$ | |
| (III-18) | $-CH_2-CH=CH_2$ | H | $CF_3$ | |
| (III-19) | $CH_3$ | H | $O-C_3H_7-n$ | |
| (III-20) | $CH_3$ | H | $O-C_3H_7-i$ | |
| (III-21) | $CH_3$ | H | $N(CH_3)_2$ | 174 |

Tabelle 6 - Fortsetzung

| Beisp. Nr. | $R^1$ | $R^3$ | $R^4$ | Schmelzpunkt [°C] |
|---|---|---|---|---|
| (III-22) | $CH_3$ | H | H | |
| (III-23) | $C_2H_5$ | H | H | |
| (III-24) | $-CH_2-CH=CH_2$ | H | H | |

Ausgangsstoffe der Formel (XIII)

Beispiel (XIII-1)

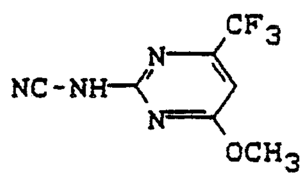

Eine Mischung aus 25,6 g (0,10 Mol) 2-Methylsulfonyl-4-trifluormethyl-6-methoxy-pyrimidin, 6,3 g (0,15 Mol) Cyanamid, 16 g (0,12 Mol) Kaliumcarbonat und 120 ml Dimethylformamid wird 48 Stunden bei 20 °C gerührt, eingeengt, mit 100 ml Wasser verrührt und mit 20 ml Essigsäure versetzt. Das kristallin angefallene Produkt wird durch Absaugen isoliert.

Man erhält 17,5 g (80 % der Theorie) 2-Cyanamino-4-trifluormethyl-6-methoxy-pyrimidin vom Schmelzpunkt 198 °C.

Analog Beispiel (XIII-1) können die in der nachstehenden Tabelle 7 aufgeführten Verbindungen der Formel (XIII) hergestellt werden.

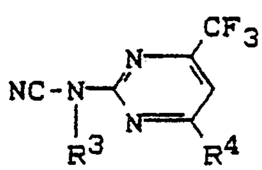

(XIII)

Tabelle 7: Beispiele für Ausgangsstoffe der Formel (XIII)

| Beisp. Nr. | $R^3$ | $R^4$ | Schmelzpunkt [° C] |
|---|---|---|---|
| (XIII-2) | H | $OC_2H_5$ | 119 |
| (XIII-3) | H | Cl | |
| (XIII-4) | H | $CH_3$ | |
| (XIII-5) | H | $CF_3$ | |
| (XIII-6) | H | $OCHF_2$ | |
| (XIII-7) | H | $OCHCF_3$ | |
| (XIII-8) | H | $NHCH_3$ | |
| (XIII-9) | H | $N(CH_3)_2$ | 197 |
| (XIII-10) | H | $O-C_3H_7-n$ | |
| (XIII-11) | H | $O-C_3H_7-i$ | |
| (XIII-12) | H | H | |

Ausgangsstoffe der Formel (XV)

Beispiel (XV-1)

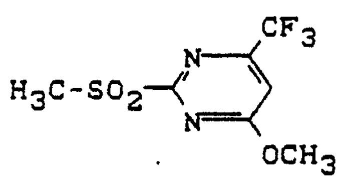

In eine Mischung aus 78 g (0,35 Mol) 2-Methylthio-4-trifluormethyl-6-methoxy-pyrimidin, 1000 ml Chloroform und 500 ml Wasser wird bei einer Innentemperatur zwischen 0 °C und +5 °C Chlor eingeleitet, bis eine bleibende Gelbfärbung einen Chlor-Überschuß anzeigt. Es wird weitere 30 Minuten bei 0 °C bis +5 °C gerührt, das überschüssige Chlor ausgeblasen, die organische Phase abgetrennt, mit Natriumsulfat getrocknet und filtriert. Das Filtrat wird eingeengt, wobei das Produkt als kristalliner Rückstand verbleibt.

Man erhält 87,6 g (98 % der Theorie) 2-Methylsulfonyl-4-trifluormethyl-6-methoxy-pyrimidin vom Schmelzpunkt 77 °C.

Analog Beispiel (XV-1) können die in der nachstehenden Tabelle 8 aufgeführten Verbindungen der Formel (XV) hergestellt werden.

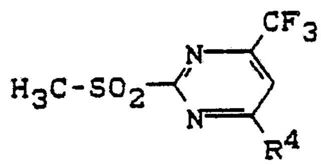

(XV)

Tabelle 8: Beispiele für Ausgangsstoffe der Formel (XV)

| Beispiel-Nr. | $R^4$ | Schmelzpunkt [° C] |
|---|---|---|
| (XV-2) | $OC_2H_5$ | 93 |
| (XV-3) | Cl | 87 |
| (XV-4) | $CH_3$ | |
| (XV-5) | $CF_3$ | |
| (XV-6) | $OCHF_2$ | |
| (XV-7) | $NHCH_3$ | |
| (XV-8) | $N(CH_3)_2$ | 169 |
| (XV-9) | $O-C_3H_7-n$ | |
| (XV-10) | $O-C_3H_7-i$ | |
| (XV-11) | H | |

## Anwendungsbeispiele

In den folgenden Anwendungsbeispielen wird die nachstehend aufgeführte Verbindung als Vergleichssubstanz herangezogen:

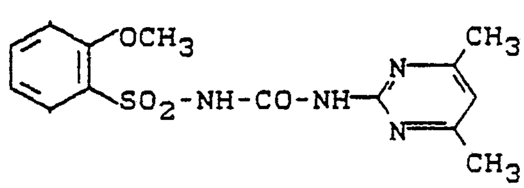

(A)

1-(2-Methoxy-phenylsulfonyl)-3-(4,6-dimethyl-pyrimidin-2-yl)-harnstoff
(bekannt aus US-PS 4 169 719).

## Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.
Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit. Beispielsweise zeigen in diesem Test die gemäß den Herstellungsbeispielen (1), (2), (6), (11), (18) und (25) erhaltenen Verbindungen im Vergleich mit der bekannten Verbindung (A) eine bessere Verträglichkeit gegenüber Kulturpflanzen, wie z. B. Weizen, und eine stärkere Wirkung gegen Problemunkräuter, wie z. B. Galium, Ipomoea und Solanum.

Beispiel B

Post-emergence-Test Lösungsmittel: 5 Gewichtsteile Aceton
Emulgator: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.
Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit. Beispielsweise zeigen in diesem Test die gemäß den Herstellungsbeispielen (1), (2), (6), (11), (18) und (25) erhaltenen Verbindungen im Vergleich mit der bekannten Verbindung (A) erheblich stärkere Wirkung gegen Problemunkräuter, wie z. B. Datura, Galium, Galinsoga, Ipomoea, Matricaria, Sinapis, Solanum und Xanthium.

**Ansprüche**

1. Substituierte 1-(2-Oxyaminosulfonyl-phenylsulfonyl)-3-(4-trifluormethyl-pyrimidin-2-yl)-(thio)harnstoffe der allgemeinen Formel

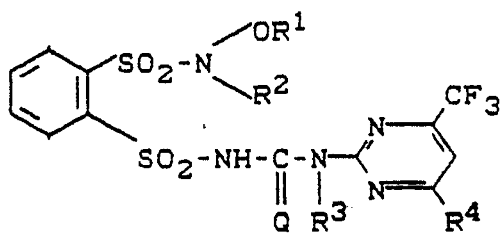

(I)

in welcher
Q für Sauerstoff oder Schwefel steht,
$R^1$ für $C_1$-$C_8$-Alkyl (welches gegebenenfalls durch Fluor, Chlor, Cyano, $C_1$-$C_2$-Alkoxy, $C_1$-$C_2$-Alkylthio, $C_1$-$C_2$-Alkylsulfinyl oder $C_1$-$C_2$-Alkylsulfonyl substituiert ist), für $C_3$-$C_6$-Alkenyl (welches gegebenenfalls durch Fluor oder Chlor substituiert ist), für $C_3$-$C_6$-Alkinyl, $C_3$-$C_6$-Cycloalkyl, $C_3$-$C_6$-Cycloalkyl-$C_1$-$C_2$-alkyl oder Phenyl-$C_1$-$C_2$-alkyl (welches gegebenenfalls durch Fluor, Chlor, Nitro, Cyano, $C_1$-$C_2$-Alkyl, $C_1$-$C_2$-Alkoxy oder $C_1$-$C_2$-Alkoxy-carbonyl substituiert ist) steht,
$R^2$ für Wasserstoff, für $C_1$-$C_8$-Alkyl (welches gegebenenfalls durch Fluor, Chlor, Cyano, $C_1$-$C_2$-Alkoxy, $C_1$-$C_2$-Alkylthio, $C_1$-$C_2$-Alkylsulfinyl, $C_1$-$C_4$-Alkylsulfonyl oder $C_1$-$C_2$-Alkoxy-carbonyl substituiert ist), für $C_3$-$C_6$-Alkenyl, $C_3$-$C_6$-Alkinyl oder Phenyl-$C_1$-$C_2$-alkyl (welches gegebenenfalls durch Fluor, Chlor, Nitro, Cyano, $C_1$-$C_2$-Alkyl, $C_1$-$C_2$-Alkoxy oder $C_1$-$C_2$-Alkoxy-carbonyl substituiert ist) steht,
oder zusammen mit $R^1$ für $C_3$-$C_5$-Alkandiyl (Alkylen) steht, oder für den Rest -CO-$R^5$ steht, worin
$R^5$ für Wasserstoff, $C_1$-$C_{10}$-Alkyl (welches gegebenenfalls durch Fluor, Chlor, Brom, Cyano, $C_1$-$C_2$-Alkoxy-carbonyl, $C_1$-$C_2$-Alkoxy, Benzyloxy oder durch gegebenenfalls durch Fluor, Chlor, Brom, Cyano, Nitro, Methyl und/oder Trifluormethyl substituiertes Phenoxy substituiert ist), $C_3$-$C_6$-Cycloalkyl, $C_3$-$C_6$-Cycloalkyl-$C_1$-$C_2$-alkyl, $C_2$-$C_6$-Alkenyl (welches gegebenenfalls durch Fluor, Chlor oder Brom substituiert ist), $C_2$-$C_6$-Alkinyl, Phenyl-$C_1$-$C_2$-alkyl (welches gegebenenfalls durch Fluor, Chlor, Nitro, Cyano, $C_1$-$C_2$-Alkyl, $C_1$-$C_2$-Alkoxy oder $C_1$-$C_2$-Alkoxy-carbonyl substituiert ist), Phenyl (welches gegebenenfalls durch Fluor, Chlor, Cyano, Nitro, $C_1$-$C_2$-Alkyl, Trifluormethyl, $C_1$-$C_2$-Alkoxy, $C_1$-$C_2$-Fluoralkoxy oder $C_1$-$C_2$-Alkoxy-carbonyl substituiert ist),.Pyridyl (welches gegebenenfalls durch Fluor, Chlor, Cyano, Nitro, $C_1$-$C_2$-Alkyl oder $C_1$-$C_2$-Alkoxy substituiert ist), Furyl (welches gegebenenfalls durch Fluor, Chlor, Cyano, Nitro, $C_1$-$C_2$-Alkyl oder $C_1$-$C_2$-Alkoxy substituiert ist), $C_1$-$C_6$-Alkoxy (welches gegebenenfalls durch Fluor, Chlor, Cyano oder $C_1$-$C_2$-Alkoxy substituiert ist), Benzyloxy (welches gegebenenfalls durch Fluor, Chlor, Nitro, Cyano, $C_1$-$C_2$-Alkyl, $C_1$-$C_2$-Alkoxy oder $C_1$-$C_2$-Alkoxy-carbonyl substituiert ist), Phenoxy (welches gegebenenfalls durch Fluor, Chlor, Cyano, Nitro, $C_1$-$C_2$-Alkyl, Trifluormethyl, $C_1$-$C_2$-Alkoxy, $C_1$-$C_2$-Fluoralkoxy oder $C_1$-$C_2$-Alkoxy-carbonyl substituiert ist) oder $C_1$-$C_2$-Alkoxy-carbonyl steht, in welcher weiter
$R^3$ für Wasserstoff, ein Alkalimetall-oder ein Erdalkalimetall-äquivalent, ein Ammonium-äquivalent, ein $C_1$-$C_4$-Alkyl-ammonium-äquivalent (welches gegebenenfalls durch Fluor, Chlor oder Hydroxy substituiert ist), ein Di-($C_1$-$C_4$)-, Tri-($C_1$-$C_4$)-oder Tetra-($C_1$-$C_4$)-alkylammonium-äquivalent oder ein Guanidinium-äquivalent, für $C_1$-$C_8$-Alkyl (welches gegebenenfalls durch Fluor, Chlor, Cyano, $C_1$-$C_2$-Alkoxy, $C_1$-$C_2$-Alkylthio, $C_1$-$C_2$-Alkylsulfinyl, $C_1$-$C_2$-Alkylsulfonyl oder $C_1$-$C_2$-Alkoxy-carbonyl substituiert ist), für $C_3$-$C_6$-Alkenyl, $C_3$-$C_6$-Alkinyl oder Phenyl-$C_1$-$C_2$-alkyl (welches gegebenenfalls durch Fluor, Chlor, Nitro, Cyano, $C_1$-$C_2$-Alkyl, $C_1$-$C_2$-Alkoxy oder $C_1$-$C_2$-Alkoxy-carbonyl substituiert ist) steht und
$R^4$ für Wasserstoff, Fluor, Chlor, Brom, $C_1$-$C_4$-Alkyl (welches gegebenenfalls durch Fluor und/oder Chlor substituiert ist), $C_1$-$C_4$-Alkoxy (welches gegebenenfalls durch Fluor, Chlor oder $C_1$-$C_2$-Alkoxy substituiert ist), $C_1$-$C_4$-Alkylamino, $C_1$-$C_4$-Alkoxyamino, N-($C_1$-$C_4$-Alkyl)-O-($C_1$-$C_4$-alkyl)-oxyamino oder Di-($C_1$-$C_2$-alkyl)-amino steht.

2. Verbindungen der Formel (I) gemäß Anspruch 1, dadurch gekennzeichnet, daß darin
Q für Sauerstoff oder Schwefel steht,
$R^1$ für $C_1$-$C_4$-Alkyl, $C_3$-$C_4$-Alkenyl oder $C_3$-$C_4$-Alkinyl steht,
$R^2$ für Wasserstoff, $C_1$-$C_4$-Alkyl, $C_3$-$C_4$-Alkenyl, $C_3$-$C_4$-Alkinyl oder
zusammen mit $R^1$ für $C_3$-$C_4$-Alkandiyl (Trimethylen oder Tetramethylen) steht, oder für den Rest -CO-$R^5$ steht, worin
$R^5$ für Wasserstoff, $C_1$-$C_4$-Alkyl (welches gegebenenfalls durch Fluor oder Chlor substituiert ist), Cyclopropyl, $C_2$-$C_4$-Alkenyl (welches gegebenenfalls durch Fluor oder Chlor substituiert ist), Benzyl, Phenyl (welches gegebenenfalls durch Fluor, Chlor, Nitro, Methyl, Trifluormethyl, Methoxy oder Trifluormethoxy substituiert ist), Pyridyl oder Furyl steht, in welcher weiter
$R^3$ für Wasserstoff, ein Natrium-, Kalium-, Calcium-oder Ammonium-äquivalent, ein $C_1$-$C_3$-Alkyl-, Di-($C_1$-$C_3$)-, Tri-($C_1$-$C_3$)-oder Tetra-($C_1$-$C_3$)-alkylammonium-äquivalent oder für Methyl steht, und
$R^4$ für Wasserstoff, Chlor, Methyl, Trifluormethyl, Methoxy, Ethoxy, Propoxy, Isopropoxy, 2,2,2-Trifluorethoxy, Difluormethoxy, Methylamino, Ethylamino oder Dimethylamino steht.

3. Verbindungen der Formel (I) gemäß Anspruch 1, dadurch gekennzeichnet, daß darin
Q für Sauerstoff steht,
$R^1$ für Methyl, Ethyl oder Allyl steht,
$R^2$ für Wasserstoff oder für den Rest -CO-$R^5$ steht, worin
$R^5$ für $C_1$-$C_3$-Alkyl (welches gegebenenfalls durch Fluor oder Chlor substituiert ist), Cyclopropyl,

Benzyl, Phenyl oder Furyl steht,
$R^3$ für Wasserstoff steht und
$R^4$ für Methoxy, Ethoxy oder Difluormethoxy steht.

4. Verfahren zur Herstellung von substituierten 1-(2-Oxyaminosulfonyl-phenylsulfonyl)-3-(4-trifluormethyl-pyrimidin-2-yl)-(thio)harnstoffen der allgemeinen Formel (I) gemäß Anspruch 1, dadurch gekennzeichnet, daß man

(a) Benzodisultame der allgemeinen Formel (II)

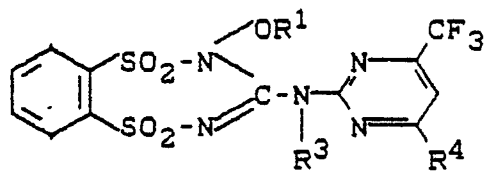

(II)

in welcher
$R^1$, $R^3$ und $R^4$ die in Anspruch 1 angegebenen Bedeutungen haben,
mit Wasser oder Schwefelwasserstoff, gegebenenfalls in Gegenwart eines Katalysators und gegebenenfalls in Gegenwart eines Verdünnungsmittels, umsetzt, oder daß man

(b) Oxyguanidine der allgemeinen Formel (III)

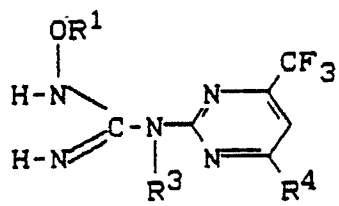

(III)

in welcher
$R^1$, $R^3$ und $R^4$ die in Anspruch 1 angegebenen Bedeutungen haben,
mit Benzol-1,2-disulfonsäure-dichlorid, gegebenenfalls in Gegenwart eines Säureakzeptors und gegebenenfalls in Gegenwart eines Verdünnungsmittels, umsetzt und die hierbei erhaltenen Benzodisultame der Formel (II) - ohne Zwischenisolierung - mit Wasser oder mit Schwefelwasserstoff, gegebenenfalls in Gegenwart eines Katalysators und gegebenenfalls in Gegenwart eines Verdünnungsmittels umsetzt, oder daß man

(c) 2-Amino-4-trifluormethyl-pyrimidine der allgemeinen Formel (IV)

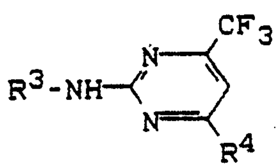

(IV)

in welcher
$R^3$ und $R^4$ die in Anspruch 1 angegebenen Bedeutungen haben,
mit 2-Oxyaminosulfonyl-phenylsulfonyl-iso(thio)cyanaten der allgemeinen Formel (V)

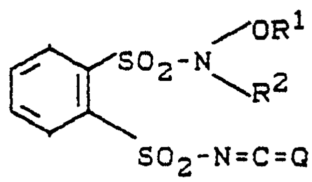

(V)

in welcher

Q, $R^1$ und $R^2$ die in Anspruch 1 angegebenen Bedeutungen haben, gegebenenfalls in Gegenwart eines Verdünnungsmittels und gegebenenfalls in Gegenwart eines Katalysators umsetzt, oder daß man

(d) 1-(2-Oxyaminosulfonyl-phenylsulfonyl)-3-(4-trifluormethyl-pyrimidin-2-yl)-(thio)harnstoffe der allgemeinen Formel (VI)

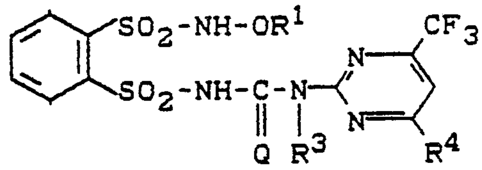

(VI)

in welcher
Q, $R^1$, $R^3$ und $R^4$ die in Anspruch 1 angegebenen Bedeutungen haben, mit Verbindungen der Formel (VII)

$R^2$ -X (VII)

in welcher
$R^2$ die in Anspruch 1 angegebene Bedeutung hat und
X für Halogen steht,
gegebenenfalls in Gegenwart eines Säureakzeptors und gegebenenfalls in Gegenwart eines Verdünnungsmittels umsetzt, und gegebenenfalls die gemäß Verfahren (a), (b), (c) oder (d) erhaltenen Verbindungen der Formel (I), in welcher $R^3$ für Wasserstoff steht, nach üblichen Methoden in Salze überführt.

5. Benzodisultame der allgemeinen Formel (II)

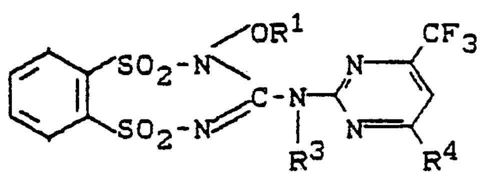

(II)

in welcher
$R^1$, $R^3$ und $R^4$ die in Anspruch 1 angegebenen Bedeutungen haben.

6. Oxyguanidine der allgemeinen Formel (III)

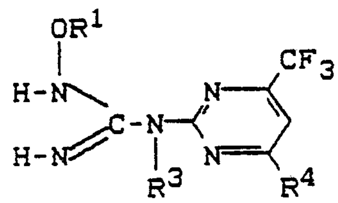

(III)

in welcher
$R^1$, $R^3$ und $R^4$ die in Anspruch 1 angegebenen Bedeutungen haben.

7. 2-Cyanamino-4-trifluormethyl-pyrimidine der allgemeinen Formel (XIII)

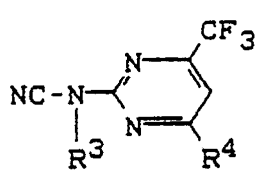

(XIII)

in welcher
$R^3$ und $R^4$ die in Anspruch 1 angegebenen Bedeutungen haben.

8. 2-Methylsulfonyl-4-trifluormethyl-pyrimidinen der allgemeinen Formel (XV)

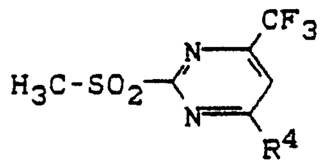

(XV)

in welcher
$R^4$ die in Anspruch 1 angegebene Bedeutung hat.

9. Herbizide Mittel, gekennzeichnet durch einen Gehalt an mindestens einem substituierten 1-(2-Oxyaminosulfonyl-phenylsulfonyl)-3-(4-trifluormethylpyrimidin-2-yl)-(thio)harnstoff der allgemeinen Formel (I) gemäß Anspruch 1.

10. Verwendung von substituierten 1-(2-Oxyaminosulfonylphenylsulfonyl)-3-(4-trifluormethyl-pyrimidin-2-yl)-(thio)harnstoffen der allgemeinen Formel (I) gemäß Anspruch 1 zur Bekämpfung von unerwünschtem Pflanzenwachstum.

11. Verfahren zur Herstellung von herbiziden Mitteln, dadurch gekennzeichnet, daß man substituierte 1-(2-Oxyaminosulfonyl-phenylsulfonyl)-3-(4-trifluormethyl-pyrimidin-2-yl)-(thio)harnstoffe der allgemeinen Formel (I) gemäß Anspruch 1 mit Streckmitteln und/oder oberflächenaktiven Mitteln vermischt.